# EUROPÄISCHE PATENTSCHRIFT

(11) **EP 3 336 099 B1**
(45) Veröffentlichungstag und Bekanntmachung des Hinweises auf die Patenterteilung: **04.03.2026**
(21) Anmeldenummer: 17002034.1
(22) Anmeldetag: 15.12.2017
(51) Int. Cl.: C07K 14/245, C12N 11/06

(54) **VERFAHREN ZUR SELEKTIVEN BINDUNG VON ZELLEN AN EINEN BINDUNGSPARTNER UND ZELLEN UMFASSENDE VORRICHTUNGEN**
METHOD FOR SELECTIVELY BINDING CELLS TO A BINDING PARTNER AND DEVICE COMPRISING CELLS
PROCÉDÉ DE LIAISON SÉLECTIVE DE CELLULES À UN PARTENAIRE DE LIAISON ET DISPOSITIFS COMPORTANT DES CELLULES

(30) Priorität: 16.12.2016 DE 102016015002
(43) Veröffentlichungstag der Anmeldung: 20.06.2018
(73) Patentinhaber: Karlsruher Institut für Technologie, 76131 Karlsruhe (DE)
(72) Erfinder: Niemeyer, Christof, 28213 Bremen (DE); Rabe, Kersten, 76133 Karlsruhe (DE); Peschke, Theo, 76131 Karlsruhe (DE)
(74) Vertreter: Müller-Boré & Partner Patentanwälte PartG mbB

(56) Entgegenhaltungen:
- MARTIN GUSTAVSSON ET AL: "Biocatalysis on the surface of Escherichia coli: melanin pigmentation of the cell exterior", SCIENTIFIC REPORTS, vol. 6, 26 October 2016 (2016-10-26), pages 36117, XP055340755, DOI: 10.1038/srep36117
- PETER Q. NGUYEN ET AL: "Programmable biofilm-based materials from engineered curli nanofibres", NATURE COMMUNICATIONS, vol. 5, 17 September 2014 (2014-09-17), GB, pages 4945, XP055302735, ISSN: 2041-1723, DOI: 10.1038/ncomms5945
- VALENCIO SALEMA ET AL: "Selection of Single Domain Antibodies from Immune Libraries Displayed on the Surface of E. coli Cells with Two &bgr;-Domains of Opposite Topologies", PLOS ONE, vol. 8, no. 9, 23 September 2013 (2013-09-23), pages e75126, XP055123932, ISSN: 1932-6203, DOI: 10.1371/journal.pone.0075126
- RICE JEFFREY J ET AL: "BACTERIAL DISPLAY USING CIRCULARLY PERMUTED OUTER MEMBRANE PROTEIN OMPX YIELDS HIGH AFFINITY PEPTIDE LIGANDS", PROTEIN SCI, WILEY, US, vol. 15, no. 4, 1 April 2006 (2006-04-01), pages 825 - 836, XP009072759, ISSN: 0961-8368, DOI: 10.1110/PS.051897806
- THEO PESCHKE ET AL: "Orthogonal Surface Tags for Whole-Cell Biocatalysis", ANGEWANDTE CHEMIE INTERNATIONAL EDITION, vol. 56, no. 8, 20 January 2017 (2017-01-20), pages 2183 - 2186, XP055462685, ISSN: 1433-7851, DOI: 10.1002/anie.201609590
- GALLUS SABRINA ET AL: "Surface Display of Complex Enzymes by in Situ SpyCatcher-SpyTag Interaction", vol. 21, no. 15, 3 August 2020 (2020-08-03), pages 2126 - 2131, XP055952591, ISSN: 1439-4227, Retrieved from the Internet <URL:https://www.ncbi.nlm.nih.gov/pmc/articles/PMC7497234/pdf/CBIC-21-2126.pdf> DOI: 10.1002/cbic.202000102
- ELEANOR M. DENHAM ET AL: "A generic cell surface ligand system for studying cell-cell recognition", PLOS BIOLOGY, vol. 17, no. 12, 9 December 2019 (2019-12-09), pages e3000549, XP055716342, DOI: 10.1371/journal.pbio.3000549

## Beschreibung

Die vorliegende Erfindung betrifft Verfahren zur selektiven Bindung von bakteriellen Zellen an ein Protein oder einen Proteinkomplex, entsprechende Zellaggregate und Bindungskomplexe, sowie deren Verwendung als Biokatalysator, Biosensor und/oder Bioreaktor.

Die selektive Immobilisierung von Zellen, insbesondere bakteriellen Zellen, auf festen Oberflächen ist von größtem Interesse für eine Reihe von Anwendungen im Bereich der industriellen Biotechnologie, wie etwa bei der Herstellung von Grundchemikalien, Bio-Pestiziden, Bio-Kunststoffen, Pharmaka oder Feinchemikalien in Durchflussreaktoren, sowie deren Online-Überwachung mit Hilfe von Biosensoren. Eine Vielzahl von entsprechenden Verfahren, basierend auf entweder dem Einschließen der Zellen in Hydrogelen oder Polymermaterialien oder der Adsorption der Zellen auf inerten porösen Trägermaterialien, wurden dabei bereits untersucht. Erstere Verfahren zielen vornehmlich auf einen Schutz der Zellen vor lebensfeindlichen Umgebungen ab, während letztere angewandt werden, wenn ein effizienter Massentransport zwischen gelösten Stoffen und den immobilisierten Zellen in heterogenen Reaktionen erforderlich ist. Dabei wurde bereits früh festgestellt, dass die Anheftung von insbesondere bakteriellen Zellen an Oberflächen äußerst günstig für kontinuierliche Bioreaktoren ist, da dies ein konstantes Wachstumsmilieu bei stabilisierten Bedingungen, hohe Zelldichten und gute Wiederverwendbarkeit für die kostengünstige Herstellung von Brennstoffen und Chemikalien ermöglicht.

Ebenso kann eine selektive Bindung von Zellen an weitere Zellen oder die selektive Bindung von Proteinen, wie etwa großen Enzymen oder Enzymkomplexen, auf Zellen von großem Nutzen, beispielsweise in der synthetischen Biologie oder Biotechnologie, sein.

Allerdings kann eine einfache chemische Immobilisierung von Zellen durch kovalente Bindung oder nicht-kovalente Adsorption zu Zellschädigungen, verringerter biologischen Aktivität und unbeständiger Anheftung der Zellen führen. Daher wurden Verfahren für eine gerichtete Immobilisierung von Zellen untersucht. Diese sollten eine effiziente und kontrollierbare Anheftung ermöglichen, was äußerst wichtig für die Anwendung als Biokatalysatoren, Biosensoren und Bioreaktoren, wie auch in der biologischen und biomedizinischen Grundlagenforschung ist.

Herkömmliche Verfahren für die gerichtete Immobilisierung von bakteriellen Zellen auf Oberflächen verlassen sich üblicherweise auf eine vorhergehende Immobilisierung spezifischer Bindemoleküle wie Antikörper oder rekombinante Zellwand-bindende Domänen, und/oder erfordern das Anfügen synthetischer Markierungseinheiten (Tags), beispielsweise Oligonukleotide, an die Zellwand. Allerdings ist das Anfügen solcher synthetischer Markierungseinheiten ein zusätzlicher, zeit- und kostenintensiver Schritt, der zusätzlichen Zellstress und die Gefahr der Abtötung der Zellen mit sich bringt. Weiter kann bei kontinuierlichen Prozessen die extern hinzugefügte Markierungseinheit über die Prozessdauer ihre Funktion verlieren. Schließlich kann die Einbettung von Zellen in Hydrogelen oder Polymermaterialien, sowie der Einschluss von Zellen in porösen Trägermaterialien, zu einer Einschränkung der Kontaktfläche zwischen den Zellen und dem umgebenden Medium führen, was wiederum die katalytische Aktivität und Prozesseffizienz herabsetzt.

In diesem Zusammenhang beschreibt Gustavsson *et al.* (Gustavsson, M. et al.; Scientific Reports, 6(26); Oktober 2016; S. 36117) biokatalytische Verfahren, die auf der Zelloberfläche von E. *coli* Zellen ablaufen. Weiter beschreibt Nguyen *et al.* (Nguyen, P. Q. et al.; Nature Communications, 5(17); September 2014; S. 4945) künstlich erzeugte Biofilme und deren Verwendung in der Biosynthese sich selbst organisierender Materialien. Weiter beschreibt Salema *et al.* (Salema, V. et al.; PLOS One, 8(9); September 2013; S. e75126) Verfahren für die Auswahl und Präsentation von Antikörper-Derivaten auf der Oberfläche von E. *coli* Zellen. Schließlich beschreibt Rice *et al.* (Rice, J. J. et al.; Protein Sci, 15(4); April 2006; S. 825-836) Verfahren für die Auswahl und Präsentation von hochaffinen Peptidliganden auf der Oberfläche von bakteriellen Zellen.

Der vorliegenden Erfindung liegt daher die Aufgabe zugrunde, Verfahren für eine einfache, effiziente, schonende und gut kontrollierbare selektive Bindung von bakteriellen Zellen an ein Protein oder einen Proteinkomplex bereitzustellen. Weiter sollen entsprechende Zellaggregate und Bindungskomplexe bereitgestellt werden, welche als Biokatalysator, Biosensor und/oder Bioreaktor verwendet werden können.

Diese Aufgabe wird durch die in den Ansprüchen gekennzeichneten Ausführungsformen gelöst. Insbesondere betrifft die vorliegende Erfindung die in den beigefügten Ansprüchen bezeichneten Ausführungsformen.

Dementsprechend betrifft ein Gegenstand der vorliegenden Erfindung ein Verfahren zur selektiven Bindung von bakteriellen Zellen an einen Bindungspartner, umfassend die Schritte:
(a) Bereitstellen von bakteriellen Zellen, wobei diese bakteriellen Zellen
   (i) auf der Zelloberfläche eine orthogonale Markierungseinheit (Tag) exprimieren und
   (ii) gleichzeitig ein funktionelles, heterologes Protein exprimieren,
(b) Bereitstellen eines Bindungspartners, der mindestens eine Art von Bindungseinheit (Bindungs-Tag) trägt, welche spezifisch an die Markierungseinheit (Tag) der bakteriellen Zellen binden kann und
(c) Inkubieren der in Schritt (a) bereitgestellten bakteriellen Zellen mit dem in Schritt (b) bereitgestellten Bindungspartner, wobei die bakteriellen Zellen über ihre Markierungseinheit an die entsprechende Bindungseinheit auf dem Bindungspartner binden,
   wobei die Markierungseinheit und Bindungseinheit so gewählt sind, dass die Markierungseinheit spezifisch und ausschließlich an die Bindungseinheit bindet, und wobei der Bindungspartner ein Protein oder Proteinkomplex ist, wobei das Protein oder zumindest ein Protein der Proteinkomplexes ein Fusionsprotein mit der Bindungseinheit darstellt, und wobei das als Bindungspartner fungierende Protein das von den zu bindenden bakteriellen Zellen exprimierte, funktionelle, heterologe Protein ist.

Der hier verwendete Begriff "selektive Bindung" bezeichnet die Tatsache, dass erfindungsgemäß definierte Typen von Zellen selektiv, also in spezifischer und gerichteter Weise, an vorab definierte Bindungspartner gebunden werden.

Die erfindungsgemäß verwendeten bakteriellen Zellen exprimieren gleichzeitig eine orthogonale Markierungseinheit (Tag) auf der Zelloberfläche und ein funktionelles Protein. Zelltypen, die dabei verwendet werden können, unterliegen keinerlei besonderen Beschränkungen, wobei *Escherichia coli* Zellen besonders bevorzugt sind.

Die erfindungsgemäß verwendeten Bindungspartner tragen mindestens eine Art von Bindungseinheit (Bindungs-Tag), welche an die Markierungseinheit der zu bindenden Zellen binden kann.

Gemäß der vorliegenden Erfindung ist der erfindungsgemäß verwendete Bindungspartner ein Protein oder Proteinkomplex, beispielsweise ein Enzym oder Enzymkomplex. In dieser Ausführungsform bezeichnet die Wendung "Bindungspartner, der mindestens eine Art von Bindungseinheit trägt" die Tatsache, dass das Protein oder zumindest ein Protein des Proteinkomplexes die Bindungseinheit mit umfasst, also mit dieser kovalent verknüpft ist. Insbesondere ist das Protein oder zumindest ein Protein des Proteinkomplexes ein Fusionsprotein mit der Bindungseinheit. Gemäß der vorliegenden Erfindung ist das als Bindungspartner fungierende Protein oder der Proteinkomplex das von den zu bindenden Zellen exprimierte funktionelle Protein. Dabei kann die Bindung der orthogonalen Markierungseinheit an die Bindungseinheit bereits intrazellulär erfolgen. Dementsprechend ist in diesem Fall Schritt (b) des erfindungsgemäßen Verfahrens bereits in der Expression des funktionellen Proteins umfasst. Weiter ist Schritt (c) des erfindungsgemäßen Verfahrens in diesem Fall bereits dann realisiert, wenn die Markierungseinheit und das die Bindungseinheit tragende funktionelle Protein durch gleichzeitige Expression im Cytosol der Zellen in räumlicher Nähe zueinander vorliegen.

In Schritt (a) des erfindungsgemäßen Verfahrens wird mindestens ein Typ von bakteriellen Zellen bereitgestellt, wobei diese Zellen (i) auf der Zelloberfläche eine orthogonale Markierungseinheit exprimieren und (ii) gleichzeitig ein funktionelles Protein exprimieren. In diesem Zusammenhang bezeichnet die Wendung "ein Typ von Zellen" eine Population von Zellen, welche die gleiche Markierungseinheit und das gleiche funktionelle Protein exprimieren.

Der Begriff "orthogonale Markierungseinheit" wie hierin verwendet bezeichnet die Tatsache, dass die erfindungsgemäßen Markierungseinheiten ausschließlich mit der jeweiligen Bindungseinheit, auch in Gegenwart anderer konkurrierender Reaktionspartner, reagieren bzw. an diese binden.

Erfindungsgemäß werden die Zellen über eine spezifische Bindung zwischen der Markierungseinheit auf der Zelloberfläche und der Bindungseinheit auf dem Bindungspartner an diesen gebunden. Entsprechende Kombinationen von Markierungseinheit und Bindungseinheit sind im Stand der Technik bekannt und unterliegen keinerlei besonderen Beschränkungen. Bevorzugte Markierungseinheit/Bindungseinheit-Paarungen umfassen (i) Streptavidin-bindendes Peptid *(streptavidin binding peptide;* SBP) und Streptavidin (STV), (ii) Biotin und eMA *(enhanced monomeric Avidin),* (iii) SpyTag (ST) und SpyCatcher (SC), (iv) SpyCatcher und SpyTag, (v) Halo-Tag oder HOB.Tag *(halo-based oligonucleotide binder)* und Chlorhexangruppen, (vi) SNAP-Tag und Benzylguaningruppen, (vii) Myc-Tag und anti-Myc-Immunglobuline, (viii) FLAG-Tag und anti-FLAG-Immunglobuline, sowie (ix) ybbR-Tag und Coenzym-A-Gruppen.

Dementsprechend umfasst die Markierungseinheit bevorzugt eines aus SBP, Biotin, SpyTag, SpyCatcher, Halo-Tag, HOB-Tag, SNAP-Tag, Myc-Tag, FLAG-Tag und ybbR-Tag. In bevorzugten Ausführungsformen ist die Markierungseinheit mittels eines flexiblen Linkers an das bakterielle Membranprotein Lpp-ompA gebunden, was eine einfache Möglichkeit zur Expression auf der Zelloberfläche der Zellen bietet. Weitere Membranproteine, welche für die Präsentation der Markierungseinheiten an der Zelloberfläche verwendet werden könne, sind im Stand der Technik bekannt. Der flexible Linker ist dabei bevorzugt ein GGGGS-Linker (SEQ ID NO: 1), wobei weitere mögliche Linker im Stand der Technik bekannt sind.

Weiter ist die Bindungseinheit bevorzugt ausgewählt aus Streptavidin, SpyCatcher, SpyTag, Chlorhexangruppen, Benzylguaningruppen, anti-Myc-Immunglobuline, anti-FLAG-Immunglobuline und Coenzym-A-Gruppen, wobei Chlorhexangruppen bevorzugt an DNA-Oligonukleotidstrukturen gebunden vorliegen, und Immunglobuline bevorzugt Antikörper, Antikörperfragmente und Antikörpermimetika, wie im Stand der Technik bekannt, umfassen.

Die Bindung zwischen Markierungseinheit und Bindungseinheit kann nicht-kovalent (wie im Falle von SBP/Streptavidin) oder kovalent (wie im Falle von SpyTag/SpyCatcher oder HOB/Chlorhexan) sein.

In bevorzugten Ausführungsformen kann die Bindung zwischen Markierungseinheit und Bindungseinheit durch Zugabe externer Reagenzien induziert und/oder aufgebrochen werden. So kann beispielsweise die Bindung zwischen ybbR-Tag und Coenzym-A-Gruppen durch das Enzym Sfp Phosphopantetheinyl Transferase vermittelt werden, oder mittels Sortase-Akzeptor (LPXTG- Motiv) und Sortase-Donor (Oligoglycin-Peptid) bzw. Sortase-Donor (Oligoglycin-Peptid) und Sortase-Akzeptorgruppen (LPXTG-Peptide) durch das Enzym Sortase verknüpft werden. Weiter kann die Bindung von Streptavidin und SBP-Tag durch die Zugabe von Biotin gelöst werden. Durch die Verwendung spezieller Biotinderivate, wie beispielsweise d-Desthiobiotin, können die Zellen reversibel abgelöst werden und eine Streptavidin-modifizierte Oberfläche durch Waschen mit HABA (2-[4-Hydroxyphenylazo]-benzoesäure) regeneriert werden, wodurch die Oberfläche wieder bereitsteht, SBP-markierte Zellen zu binden. Werden zwischen das Membranprotein und die Markierungseinheit Protease-Schnittstellen eingefügt, können die Zellen durch Zugabe einer Protease wieder von der Bindungseinheit gelöst werden. Des Weiteren kann die Bindungseinheit beispielsweise auch photolabile oder chemisch labile Gruppen enthalten und so vom Trägermaterial gelöst werden.

Das funktionelle Protein, welches von den Zellen gleichzeitig mit der Markierungseinheit exprimiert wird, unterliegt keinerlei besonderen Beschränkungen und umfasst jegliche Proteine, die im Zusammenhang mit der jeweiligen Anwendung oder Fragestellung von Interesse sind. In diesem Zusammenhang bezeichnet "gleichzeitige Expression" die Tatsache, dass das funktionelle Protein und die Markierungseinheit zur gleichen Zeit auf und/oder in den Zellen vorhanden ist. Funktionelle Proteine können homologe oder heterologe Proteine sein, und können entweder cytosolisch oder auf der Zelloberfläche exprimiert werden. In bevorzugten Ausführungsformen ist das funktionelle Protein ein Enzym, etwa ein stereoselektives Enzym, und/oder ein Enzym das ausgewählt ist aus der Gruppe, bestehend aus Lipasen, Glycosidasen, Ketoreduktasen, Oxidoreduktasen, Polymerasen, Hydrolasen und Ligasen. Weiter kann das funktionelle Protein in bevorzugten Ausführungsformen ein Protein mit einer bestimmten nachweisbaren spektroskopischen Eigenschaft sein. Dies können insbesondere fluoreszierende Proteine oder Proteine sein, welche eine Biolumineszenz hervorrufen, wie dies beispielsweise bei den Luciferasen geschieht. Ebenfalls bevorzugt sind Proteine mit einer bestimmten nachweisbaren spektroskopischen Eigenschaft, die als Sensoren für die Bindung von Analytmolekülen genutzt werden können. Dies sind beispielsweise Fusionsproteine, die neben fluoreszierenden Proteindomänen eine Bindungsstelle für einen Analyten enthalten und die Bindung des Analyten durch Änderung der spektroskopischen Eigenschaft anzeigen. Im Stand der Technik bekannte Beispiele in diesem Zusammenhang sind Proteinbiosensoren auf Basis des Glukose- oder Galaktose-Bindungsproteins. Weiterhin bevorzugt sind Proteine, die spezifisch Metallionen, Metalloxide und/oder keramische Oxidfragmente binden können. Entsprechende Beispiele umfassen das Goldbindungsprotein, Ferritin, und Silicabindungspeptide und -proteine. Dem Fachmann sind eine Vielzahl solcher Proteine bekannt, die üblicherweise für Anwendungen in der Bioremedation verwendet werden. Darüber hinaus können Proteine Verwendung finden, die an chemische (z.B. Polyethylen, Polypropylen) oder biologische Polymere (z.B. Cellulose, Chitin) binden können.

Bevorzugt werden das funktionelle Protein und die Markierungseinheit unabhängig voneinander auf verschiedenen genetischen Vektoren kodiert. Dies umfasst die Verwendung von orthogonal induzierbaren Plasmiden, also Plasmiden, welche die spezifische und selektive Induktion der Expression von Markierungseinheit und funktionellem Protein unabhängig voneinander durch geeignete Induktoren ermöglichen. Dies kann beispielsweise die Verwendung von Arabinose- und IPTG-induzierbaren Promotoren umfassen.

Weiter kann die Expression von Markierungseinheit und funktionellem Protein in anderen Ausführungsformen entweder mit demselben oder mit verschiedenen induzierbaren Promotoren angestoßen werden. Ebenso können die Markierungseinheit und das funktionelle Protein in bestimmten Ausführungsformen auf nur einem Plasmid kodiert und/oder genomisch integriert vorliegen. Dementsprechend kann monocistronische und/oder polycistronische mRNA gebildet werden.

In Schritt (b) des erfindungsgemäßen Verfahrens wird der vorstehend bereits definierte Bindungspartner bereitgestellt, welcher mindestens eine Art der ebenso bereits vorstehend definierten Bindungseinheiten trägt. Die Bindungseinheit kann dabei mittels kovalenter oder nicht-kovalenter Bindung auf dem Bindungspartner gebunden sein.

In Schritt (c) des erfindungsgemäßen Verfahrens werden die in Schritt (a) bereitgestellten Zellen mit dem in Schritt (b) bereitgestellten Bindungspartner inkubiert, wobei die Zellen über ihre Markierungseinheit an die entsprechenden Bindungseinheit auf dem Bindungspartner binden und so, im Falle eines festen Trägermaterials, auf diesem immobilisiert werden. Bedingungen hinsichtlich Dauer der Inkubation, Temperatur und Pufferbedingungen, bei welchen diese Bindung bzw. Immobilisierung erfolgen kann, sind im Stand der Technik bekannt.

Ein weiterer Gegenstand der vorliegenden Erfindung betrifft Zellaggregate, umfassend:
(a) bakterielle Zellen, welche mindestens eine Art von Bindungseinheit (Bindungs-Tag) tragen und
(b) bakterielle Zellen, wobei diese bakteriellen Zellen
   (i) auf der Zelloberfläche eine orthogonale Markierungseinheit (Tag) exprimieren und
   (ii) gleichzeitig ein funktionelles, heterologes Protein exprimieren,

wobei die bakteriellen Zellen aus (b) mittels spezifischer Bindung ihrer Markierungseinheit an die entsprechende Bindungseinheit auf den bakteriellen Zellen aus (a) an diese gebunden sind,
wobei die bakteriellen Zellen aus (a) identisch mit den bakteriellen Zellen aus (b) sind, wobei die Markierungseinheit und Bindungseinheit so gewählt sind, dass die Markierungseinheit spezifisch und ausschließlich an die Bindungseinheit bindet, und
wobei der Bindungspartner ein Protein oder Proteinkomplex ist, wobei das Protein oder zumindest ein Protein der Proteinkomplexes ein Fusionsprotein mit der Bindungseinheit darstellt, und wobei das als Bindungspartner fungierende Protein das von den zu bindenden bakteriellen Zellen exprimierte, funktionelle, heterologe Protein ist.

In bevorzugten Ausführungsformen der erfindungsgemäßen Zellaggregate treffen alle vorstehend für das erfindungsgemäße Verfahren genannten Einschränkungen, Definitionen und Ausführungsformen in analoger Weise auf die erfindungsgemäßen Zellaggregate zu. Insbesondere sind die Zellen als Bindungspartner, die Bindungseinheiten, die zu bindenden Zellen, die orthogonalen Markierungseinheiten und die funktionellen Proteine bevorzugt wie vorstehend für das erfindungsgemäße Verfahren definiert.

Bevorzugt sind die erfindungsgemäßen Zellaggregate durch das erfindungsgemäße Verfahren erhältlich.

Ein weiterer Gegenstand der vorliegenden Erfindung betrifft Bindungskomplexe, umfassend:
(a) Proteine oder Proteinkomplexe, welche mindestens eine Art von Bindungseinheit (Bindungs-Tag) tragen und
(b) bakterielle Zellen, wobei diese bakteriellen Zellen
   (i) auf der Zelloberfläche eine orthogonale Markierungseinheit (Tag) exprimieren und
   (ii) gleichzeitig ein funktionelles Protein exprimieren,

wobei die bakteriellen Zellen mittels spezifischer Bindung ihrer Markierungseinheit an die entsprechende Bindungseinheit auf den Proteinen oder Proteinkomplexen an diese gebunden sind,
wobei die Markierungseinheit und Bindungseinheit so gewählt sind, dass die Markierungseinheit spezifisch und ausschließlich an die Bindungseinheit bindet, und
wobei der Bindungspartner ein Protein oder Proteinkomplex ist, wobei das Protein oder zumindest ein Protein der Proteinkomplexes ein Fusionsprotein mit der Bindungseinheit darstellt, und wobei das als Bindungspartner fungierende Protein das von den zu bindenden bakteriellen Zellen exprimierte, funktionelle, heterologe Protein ist.

In bevorzugten Ausführungsformen der erfindungsgemäßen Bindungskomplexe treffen alle vorstehend für das erfindungsgemäße Verfahren genannten Einschränkungen, Definitionen und Ausführungsformen in analoger Weise auf die erfindungsgemäßen Bindungskomplexe zu. Insbesondere sind die Zellen als Bindungspartner, die Bindungseinheiten, die zu bindenden Zellen, die orthogonalen Markierungseinheiten, die funktionellen Proteine und die Proteine oder Proteinkomplexe bevorzugt wie vorstehend für das erfindungsgemäße Verfahren definiert.

Bevorzugt sind die erfindungsgemäßen Bindungskomplexe durch das erfindungsgemäße Verfahren erhältlich.

Ein weiterer Gegenstand der vorliegenden Anmeldung betrifft die Verwendung der erfindungsgemäßen Zellaggregate und/oder der erfindungsgemäßen Bindungskomplexe als Biokatalysator und/oder Biosensor und/oder Bioreaktor. Dabei werden die von den Zellen exprimierten funktionellen Proteine gemäß der jeweiligen Anwendung oder Fragestellung ausgewählt.

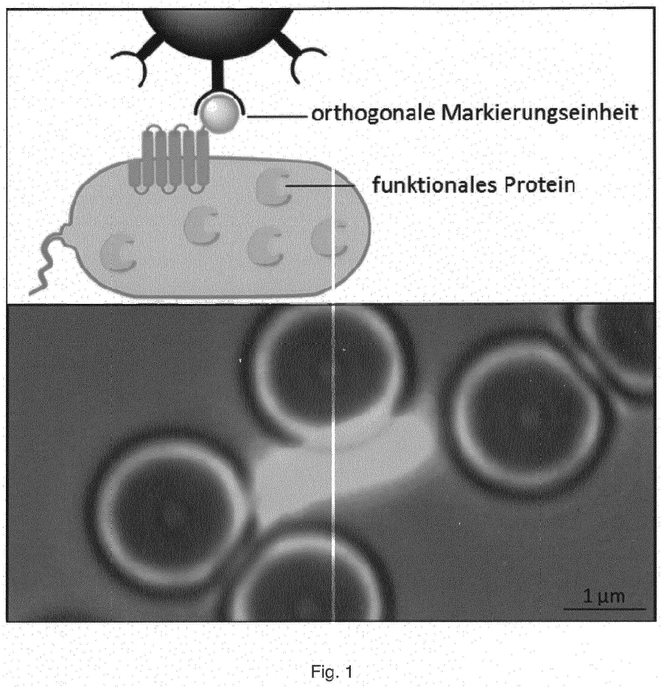

Die vorliegende Erfindung beruht auf der Präsentation von Markierungseinheiten an der Oberfläche von Zellen. Dies stellt einen eleganten Weg zur Verfügung, Zellen gerichtet auf Oberflächen zu immobilisieren, bzw. an andere Zellen oder an Proteine oder Proteinkomplexe zu binden, wobei diese Zellen zusätzlich ein je nach Anwendung oder Fragestellung frei wählbares funktionelles Protein exprimieren (Fig. 1). Dies können beispielsweise Enzyme sein, die eine gewünschte Reaktion katalysieren, so dass die Zellen als Ganzzellbiokatalysatoren funktionieren. Entsprechende Trägermaterialien, Zellaggregate oder Bindungskomplexe können so als Biokatalysator oder Bioreaktor verwendet werden, beispielsweise in der effizienten, schonenden und kostengünstigen biokatalytischen Synthese von Feinchemikalien oder Biobrennstoffen. Die Zellen können dabei von Anwendern mit wenig Vorwissen kultiviert und beispielsweise auf Oberflächen immobilisiert werden. Die eigentliche Immobilisierung oder Bindung erfolgt dabei aufgrund der erfindungsgemäßen Verwendung von Markierungseinheiten und Bindungseinheiten selbstorganisiert. In Verbindung mit Bioprinting-Verfahren können weiter beliebige Anordnungen, Muster, Arrays oder Zellverbände eines einzelnen Typs von Zellen oder von mehreren Typen von Zellen erzeugt werden.

Die Figuren zeigen:
- Figur 1:: Schematische Darstellung (A) einer E. *coli* Zelle, die orthogonale Markierungseinheiten für die Immobilisierung auf der Zelloberfläche präsentiert und gleichzeitig heterologe funktionelle Proteine im Cytosol überexprimiert. Die mikroskopische Aufnahme (B) zeigt eine bakterielle Zelle, welche eGFP *(enhanced green fluorescent protein)* exprimiert (grünes Stäbchen) und SBP auf ihrer Oberfläche präsentiert, um die Bindung an mit Streptavidin beschichtete magnetische Mikropartikel zu ermöglichen.

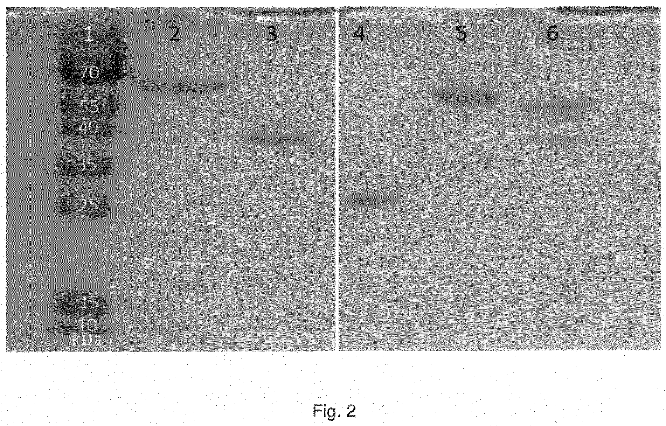

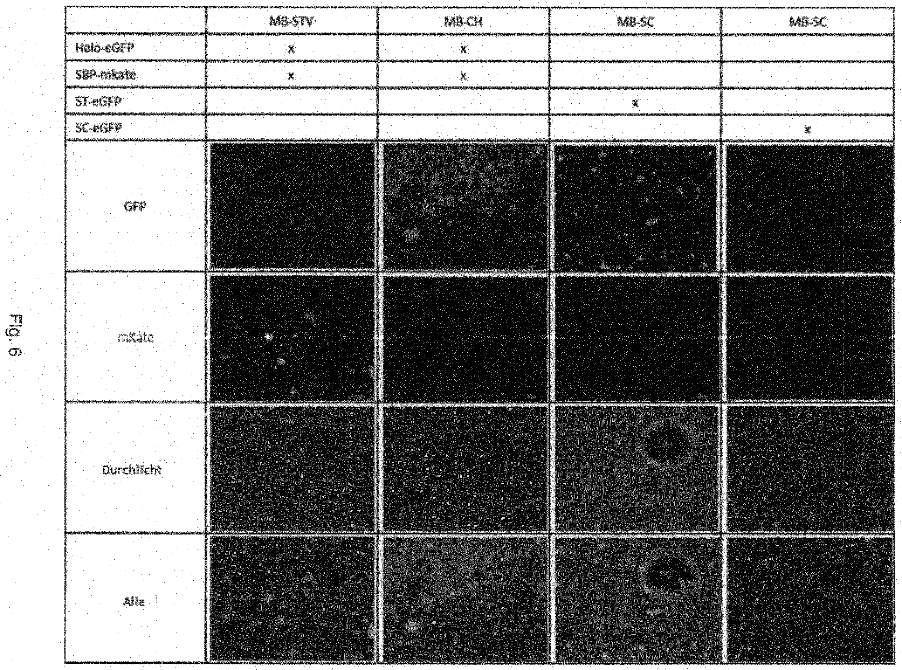

- Figur 2:: Charakterisierung von Fluoreszenz-markierten Proteinsonden, welche verwendet werden, um die Bindungsspezifität von an der Oberfläche präsentierten Markierungseinheiten und die Funktionalität der magnetischen Mikropartikel (vgl. Figur 6) zu bestimmen. Gezeigt ist eine in 16% Coomassie gefärbte SDS-PAGE. Spur 1: PageRuler Prestained Protein Ladder (Thermo Scientific); Spur 2: Cy3-STV (60 kDa); Spur 3: SC-eGFP (40,2 kDa); Spur 4: ST-eGFP (29,5 kDa); Spur 5: Halo-eGFP (63,2 kDa); Spur 6: SBP-mKate (52,9 kDa).

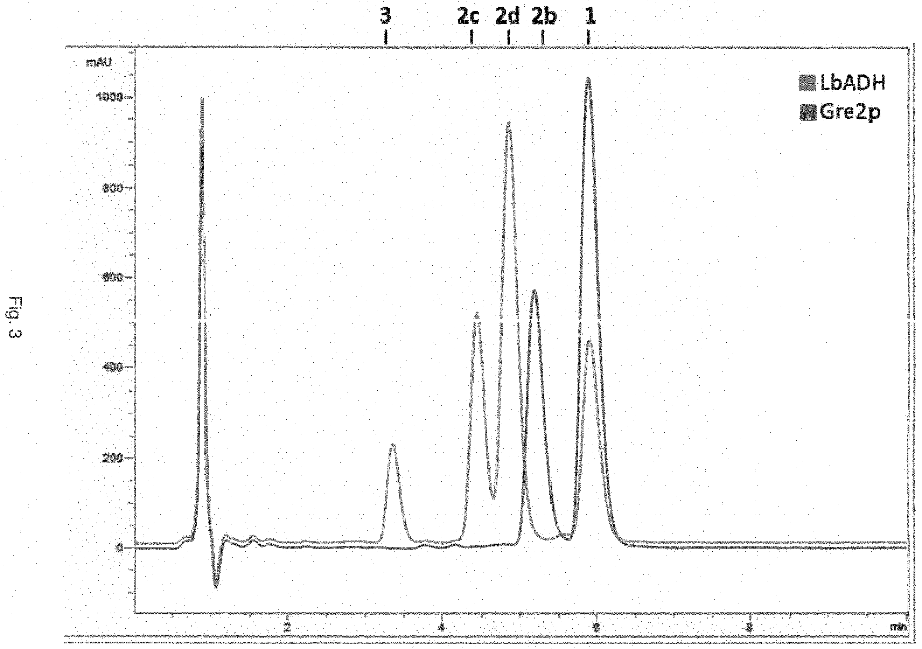

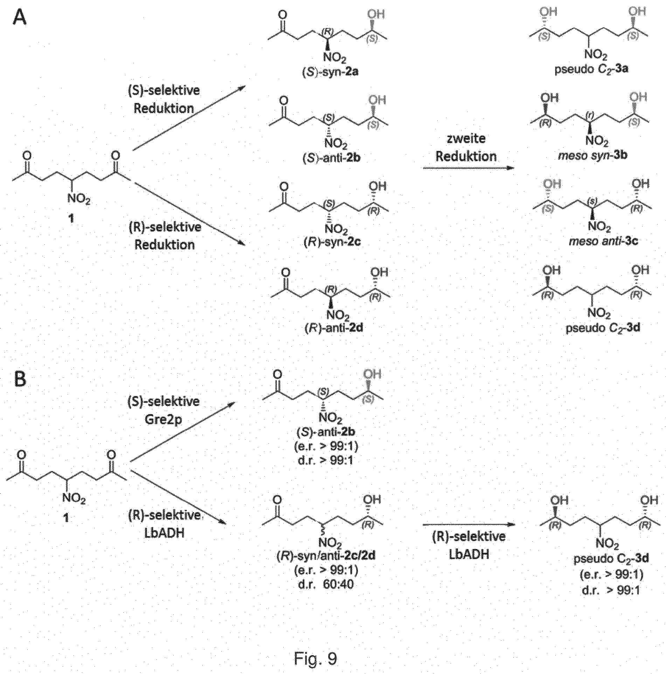

- Figur 3:: Beispielhafte chirale HPLC-Chromatogramme, welche die Trennung von verschiedenen chiralen Reduktionsprodukten eines symmetrischen Diketons (NDK 1, 5-Nitrononan-2,8-dion), hergestellt durch die Enzyme LbADH (rot) und Gre2p (blau), zeigen. Die Nummern der verschiedenen Stereoisomere entsprechen denen aus Figur 9.

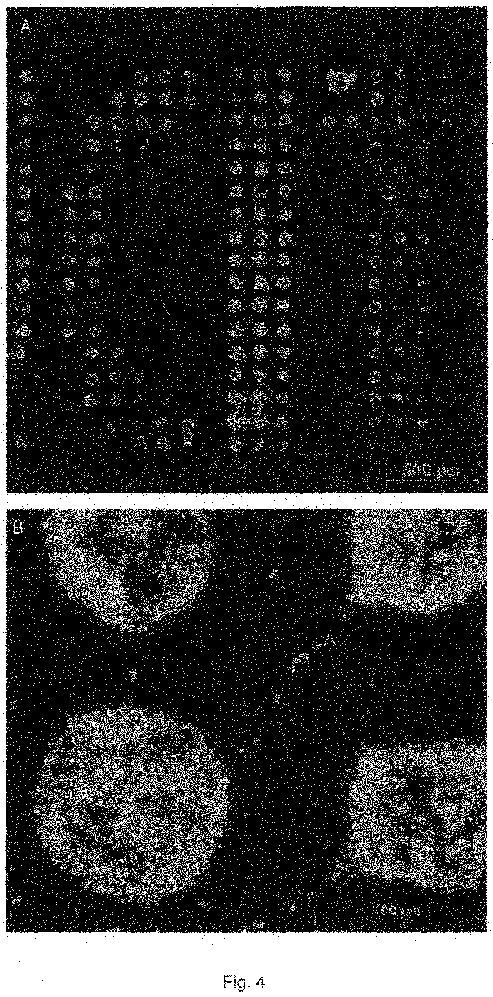

- Figur 4:: Anordnung von mKATE@E.coli-SBP in Form eines Musters auf einer Oberfläche unter Verwendung eines Proteinarrays, welches durch Tintenstrahldruck von 10 µM Streptavidin in einem Trehalosepuffer (200 mM K₂HPO₄, 200 mM KH₂PO₄, 0,5% Trehalose Dehydrat) auf chemisch aktivierte Bisepoxypolyethylenglycol (EPEG)-Objektträger hergestellt wurde. Nach dem Druck wurden die Objektträger kurz mit Blockierungspuffer (1 M Glycin, 100 mM KPᵢ pH=7,5) gewaschen, in kleine Petrischalen gelegt und mit einer Suspension von mKATE@E.coli-SBP in Medium überschichtet. Die Zellen wurden mittels Zentrifugation in einem Ausschwingrotor bei 1000 g für 10 Minuten auf die Glasoberfläche sedimentiert. Nachfolgend wurden die Objektträger mit 100 mM KPᵢ-Puffer pH=7,5 gewaschen. Aufnahmen erfolgten mit einem Zeiss Axiovert 200M Fluoreszenzmikroskop. Die gezeigten Aufnahmen bei 2,5-facher (A) und 40-facher (B) Vergrößerung zeigen deutlich die spezifische Bindung der bakteriellen Zellen an den mit Streptavidin funktionalisierten Stellen.

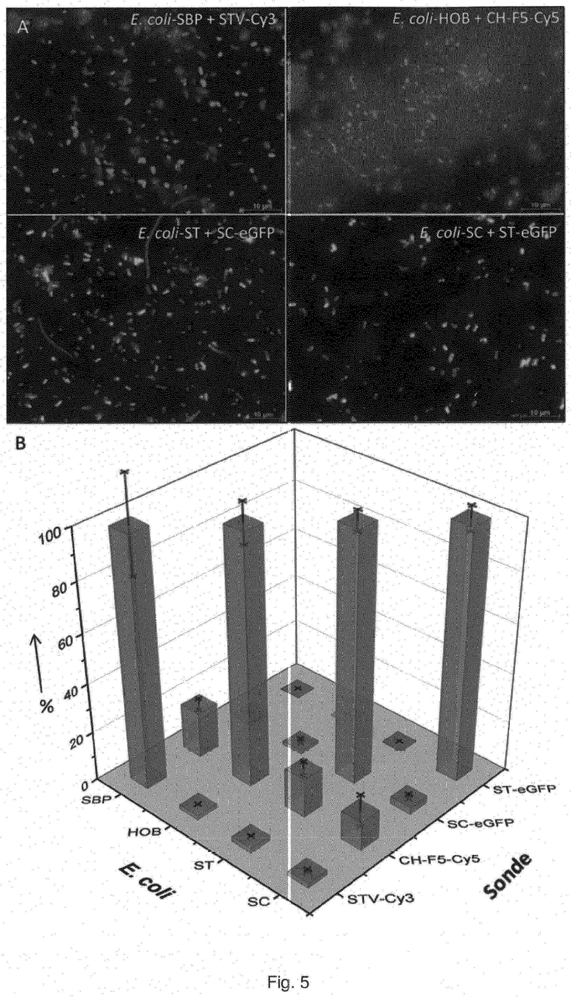

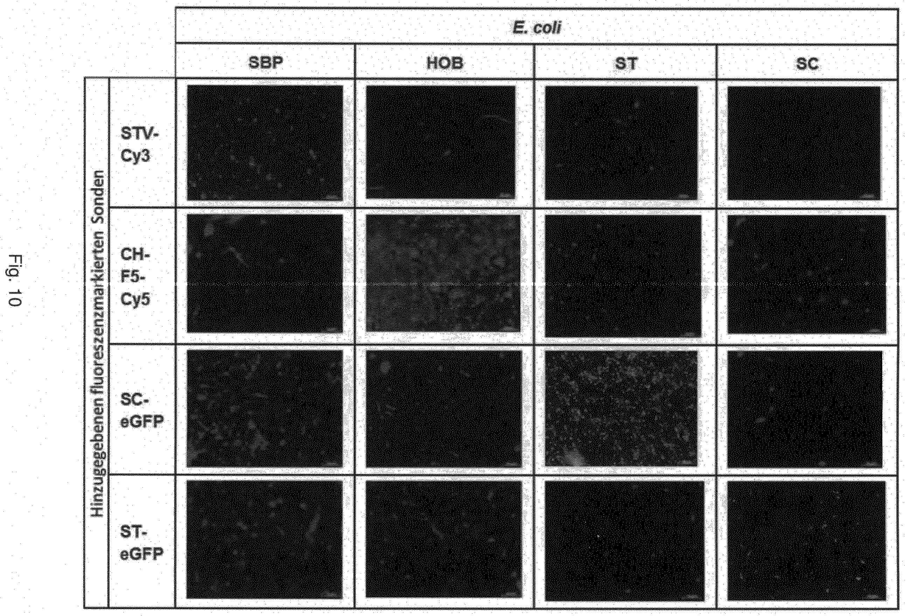

- Figur 5:: Selektive Bindung fluoreszierender biomolekularer Sonden durch E. *coli* Zellen, welche die entsprechenden Markierungseinheiten an ihrer Oberfläche präsentieren. Jeder der vier verschiedenen Zelltypen wurde mit den vier verschiedenen Sonden inkubiert. Nach Entfernen der ungebundenen Sonden wurde die verbleibende Fluoreszenz der Zellen bestimmt. (A) Repräsentative Fluoreszenzaufnahmen von E. *coli* Zellen, die mit ihrer spezifischen komplementären Sonde markiert wurden (blau: DAPI-gefärbte Bakterien; rot: STV-Cy3; violett: CH-F5-Cy5; grün: SC/ST-eGFP). Maßstabsleiste 10 µm. (B) Quantitative fluorimetrische Daten. Die Säulen zeigen relative Fluoreszenzeinheiten *(relative fluorescence units;* RFU) pro Menge an Zellen, normalisiert auf das höchste Fluoreszenzsignal innerhalb jedes Sondensatzes (vgl. Figuren 2 und 10 für die Charakterisierung der Sonden und zusätzliche Fluoreszenzaufnahmen).
- Figur 6:: Funktionalitätstest der Oberflächen der Mikropartikel. Fluoreszierende Proteine, die mit einer Markierungseinheit versehen sind, werden selektiv auf magnetischen Partikeln immobilisiert, welche die entsprechenden Bindungspartner präsentieren. Gezeigt werden repräsentative mikroskopische Aufnahmen, welche mit Filtersätzen für eGFP, mKate oder mittels Differentialinterferenzkontrast *(differential interference contrast;* DIC) aufgenommen wurden. Maßstabsleiste 20 µm. Bemerkenswerterweise bindet Halo-eGFP selektiv an magnetische Partikel, die an ihrer Oberfläche mit einer Chlorhexylgruppe (CH) funktionalisiert wurden (MB-CH). Das rot fluoreszierende Protein SBP-mKate bindet an mit Streptavidin beschichtete magnetische Partikel (MB-STV). Mikropartikel, die das SC-Protein auf ihrer Oberfläche tragen (MB-SC), können wie erwartet die ST-eGFP-, aber nicht die SC-eGFP-Sonden binden. Diese Ergebnisse zeigen, dass die drei orthogonalen Interaktionspartner funktionell auf der Oberfläche der Kügelchen vorliegen und mit ihren entsprechenden Markierungseinheiten auf spezifische Weise interagieren können.

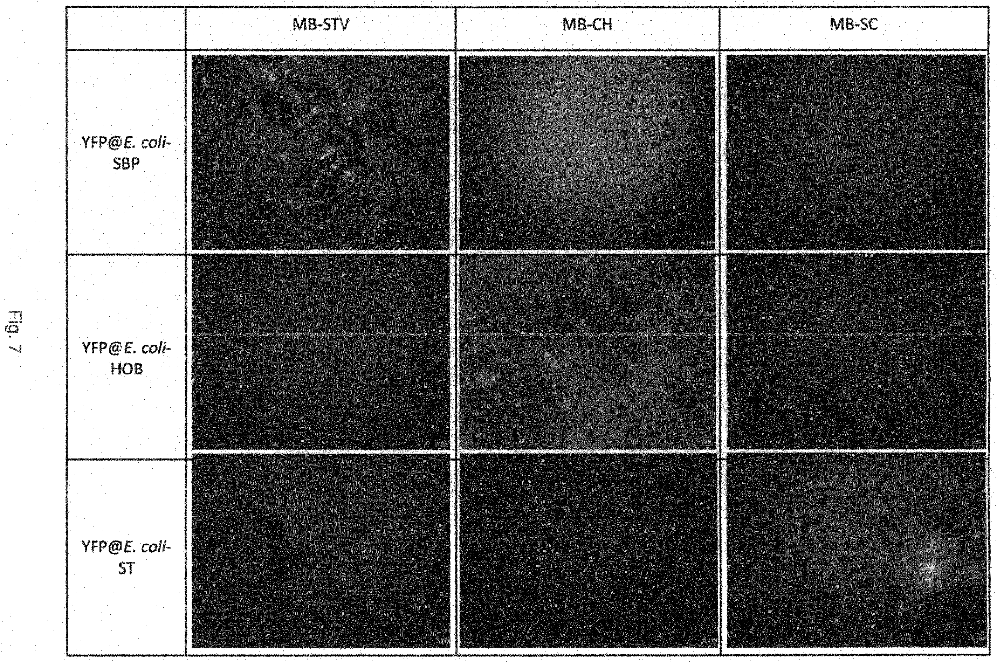

- Figur 7:: Zellen, welche eine Markierungseinheit auf ihrer Oberfläche präsentieren und gleichzeitig das fluoreszierende Protein YFP als "funktionellen Inhalt" in ihrem Cytosol exprimieren, können selektiv auf Mikropartikeln immobilisiert werden, welche die entsprechenden Interaktionspartner tragen. Die drei verschiedenen Zelltypen beinhalten zwei orthogonale Plasmide, welche ein Fusionsprotein des Membranproteins Lpp-ompA und der jeweiligen Markierungseinheit (SBP, HOB oder ST), sowie das YFP (sichtbar als grüne Strukturen), kodieren. Selektive Bindung an die Kügelchen führt zu der Bildung von Zell-Kügelchen-Aggregaten. Maßstabsleiste 5 µm.

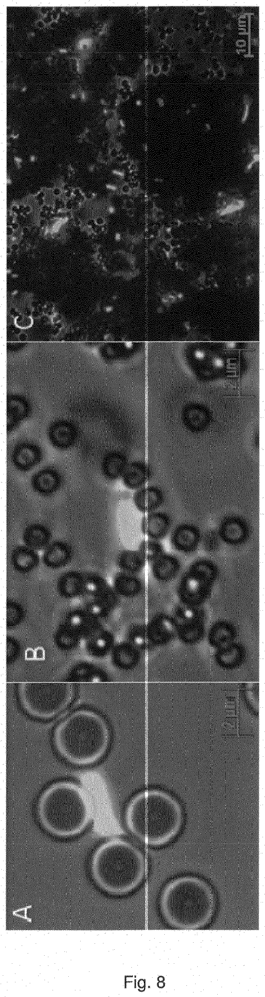

- Figur 8:: Fluoreszenzmikroskopische Aufnahmen von an mit STV funktionalisierte 2,7 µm Dynabeads M270-Streptavidin (A) und 1 µm Dynabeads C1-Streptavidin (B, C) gebundene YFP@E.coli-SBP.
- Figur 9:: (A) Die sequentielle biokatalytische Reduktion von 5-Nitrononan-2,8-dion (NDK) **1** erlaubt die selektive Darstellung stereoisometrischer Hydroxyketone **2** und Diole **3.** (B) Die beiden in dieser Studie verwendeten Modellenzyme LbADH und Gre2p führen jeweils zur selektiven Bildung der Hydroxyketone **2b** oder **2c/2d.** Während Gre2p Hydroxyketone nicht als Substrat akzeptiert, kann LbADH die Hydroxyketone **2c/2d** reduzieren, um das Pseudo-C₂-Diol **3d** zu erzeugen. Alle Stereoisomere können durch chirale HPLC-Analyse quantifiziert werden (Fig. 4).
- Figur 10:: Fluoreszierende biomolekulare Sonden werden selektiv auf der Oberfläche von E. *coli* Zellen immobilisiert, welche die jeweiligen Markierungseinheiten präsentieren. Um die Zugänglichkeit und Selektivität der an der Oberfläche präsentierten Markierungseinheiten zu untersuchen, wurde es identischen Mengen an Zellen ermöglicht, an die Fluoreszenz-markierten Sonden zu binden. Nach Inkubation für eine Stunde wurden ungebundene Sonden durch Zentrifugation/Resuspendierung entfernt und die verbleibende Fluoreszenz auf den Zellen mittels eines Fluoreszenz-Mikroplatten-Lesegeräts quantifiziert, um die in Figur 5B gezeigten Daten zu erhalten. Aliquots der Zellen wurden mit DAPI gefärbt und mittels Fluoreszenzmikroskopie analysiert. Die gezeigten repräsentativen Fluoreszenzaufnahmen wurden mit 40- facher Vergrößerung aufgenommen (blau: DAPI-gefärbte Bakterien; rot: STV-Cy3; violett: CH-F5-Cy5; grün: SC/ST-eGFP), Maßstabsleiste 10 µm. Bemerkenswerterweise sind Cy3-, Cy5- und eGFP-Fluoreszenzsignale nur auf Kügelchen sichtbar, die mit den komplementären Liganden beschichtet sind.

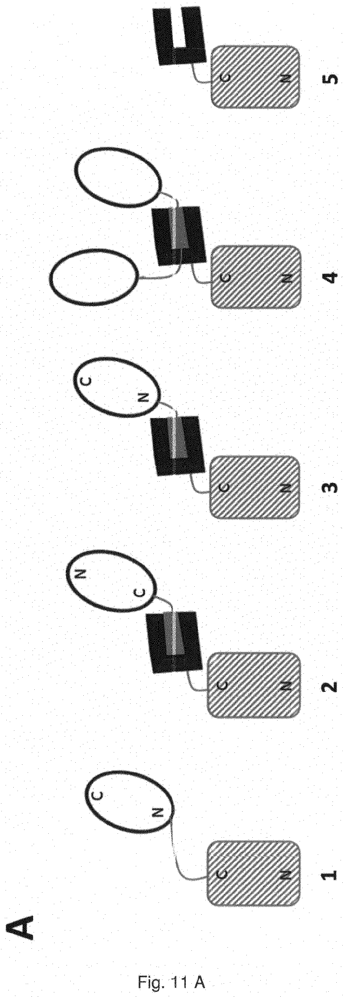

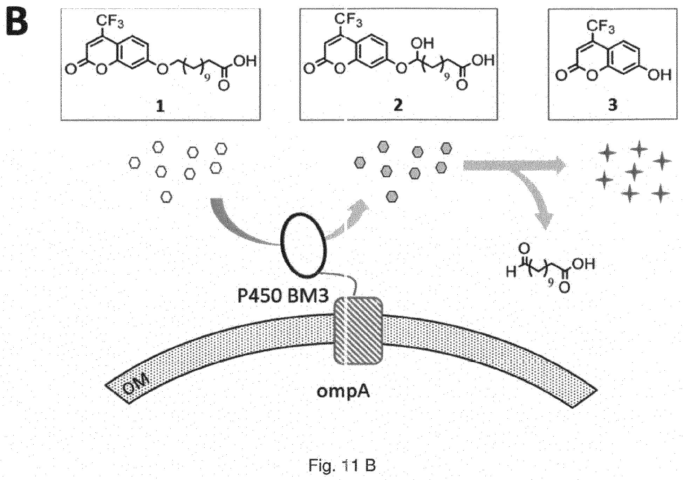

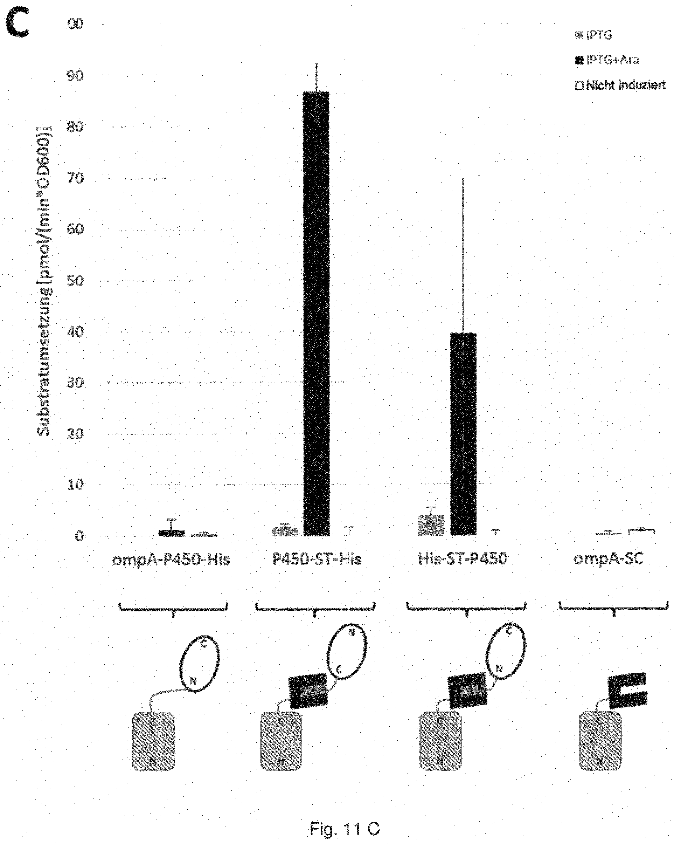

- Figur 11:: (A) Die Oberflächenexposition von großen, komplexen Zielproteinen und -peptiden (weißes Oval) lässt sich als direkte Fusion mit einem Membranprotein (grau schraffiertes Rechteck) realisieren **(1).** Durch die beschriebene getrennte Expression lassen sich Zielproteine und -peptide auch C- oder N-terminal **(2** und **3),** sowie internal **(4)** markieren (dunkelgrau) und mit dem Membranprotein, welches mit einer Bindeeinheit (schwarz) genetisch fusioniert vorliegt **(5),** an der Zelloberfläche exponieren. Bei dem Fall der internalen Markierung des Zielproteins **(4)** bleiben dessen N und C-terminale Enden in ihrem nativen Zustand. Die Zielproteine und -peptide können mittels der Verfahren **2, 3** und **4** bereits in der Zelle unabhängig vom Membranprotein gefaltet und mit Modifikationen versehen werden.
(B) Im gezeigten Beispiel werden Zellen analysiert, die wie in (A) beschrieben eine Oxidoreduktase (hier: BM3 P450 A74G F87V) exprimieren. Dieses Enzym besteht aus zwei Domänen, die ein Häm- bzw. ein Flavinnukletoid-Molekül als Kofaktor enthalten. Das BM3 Enzym kann in einer Redoxreaktion das Substrat **4,** durch Oxidation des Cofaktors NADPH, hydroxylieren. Das entstehende Zwischenprodukt **5** hydrolysiert und bildet das fluoreszente Produkt **6.** Da das Substrat **4** nicht in die Zellen eintreten kann, wird nur enzymatische Aktivität detektiert, die sich auf der Zelloberfläche befindet. Zur Vereinfachung ist hier das BM3 als weißes Oval symbolisiert. OM = outer membrane, ompA = outer membrane protein A.
(C) Substratumsetzung pro Zelldichte der in (A) beschriebenen Zelltypen. Signifikante Aktivität wird nur in **A2** und **A3** detektiert, worin anstelle der Direktfusion **(A1)** die getrennte Expression von Membranprotein und Zielprotein und anschließende intrazelluläre Kupplung der beiden Komponenten verwendet wird. Ebenso wird bei den Zellen, die lediglich das Membranprotein exprimieren **(A5),** erwartungsgemäß keine enzymatische Aktivität beobachtet. Eine Induktion mit Arabinose (Ara) führt zur Expression der Membranproteinfusionen auf den Zelloberflächen, Induktion mittels IPTG führt zur Expression des Zielproteins mit Bindeeinheit (hier P450 BM3 A74G F87V mit (ST) SpyTag).

Die vorliegende Erfindung wird anhand der folgenden, nicht-einschränkenden Beispiele näher erläutert.

### Beispiele

### Material und Methoden:

### Klonierung der Plasmide.

Die Klonierung der Plasmide basierte auf bekannten Expressionsvektoren für die löslichen Proteine Gre2p, eYFP und Halo-eGFP. Soweit nicht anders angegeben wurden die Plasmide unter Verwendung isothermaler Rekombination mit Oligonukleotidprimern mit homologen Überlappungen von 30 bp konstruiert. Alle in dieser Studie verwendeten Primer sind in der nachfolgenden Tabelle 1 angegeben. Nach dem Zusammenbau wurden die Reaktionsmischungen mit Dpn I behandelt, um verbleibende Vektoren aus früheren PCR-Reaktionen zu entfernen, und dann in E. *coli* DH5α Zellen transformiert. Alle Plasmide wurden mit ZR Plasmid Miniprep-Classic (Zymo Research, Deutschland) gemäß Herstellerangaben gereinigt und die Sequenzen durch kommerzielle Sequenzierung (LGC Genomics, Deutschland) überprüft.

**Tabelle 1: In der vorliegenden Studie verwendete Primersequenzen**

| Bezeichnung | Primersequenz | SEQ ID NO |
|---|---|---|
| TP1 | TTCCAGTTCACCAGCCAGACCTTCAACAACGTGACCACCACGCCAACCGGTG GTTTTTTCGTCCATGCTACCACCACCACCCACACCATTATCC | 2 |
| TP2 | CTGGTGAACTGGAACAGCTGCGTGCTCGTCTGGAACACCACCCGCAGGGTCA GCGTGAACCCTAATGAGATCCGGCTGCTAACAAAGCCCGAAAG | 3 |
| TP3 | T GAGAT CCGGCT GCTAACAAAGCCCGAAAG | 4 |
| TP4 | GCTACCACCACCACCCACACCATTATCC | 5 |
| TP5 | CCGGATAATGGTGTGGGTGGTGGTGGTAGCGTTGATACCCTGAGCGGTCTGA G | 6 |
| TP6 | TCGGGCTTTGTTAGCAGCCGGATCTCATTAAATATGTGCATCACCTTTGGTT GCTTTAC | 7 |
| TP7 | TATAGGCATCAACCATAACAATATGTGCATGTATATCTCCTTCTTAAAGTTA AACAAAATTA | 8 |
| TP8 | TTATGGTTGATGCCTATAAACCGACCAAAGGTGGTGGTGGCAGCGTTAG | 9 |
| TP11 | GGATGATTCTGGTAACAGGGAATGTGATTGAATTTTGTTTAACTTTAAGAAG GAGATATAC | 10 |
| TP12 | CTAACACCGAAAGAGGCTTTTCGCACGGTTGCAAAAAACCCCTCAAGACC | 11 |
| TP13 | AACCGTGCGAAAAGCCTC | 12 |
| TP14 | CAATCACATTCCCTGTTACCAG | 13 |
| 41_KSR23_for | GATCCGGCTGCTAACAAAGC | 14 |
| 42_KSR23_rev | GTTAAACAAAATTATTTCTAGAGGGGAATTG | 15 |
| 124_KSR65_for | GATCCGGCTGCTAACAAAGCCCG | 16 |
| 125_KSR65_rev | ATGTATATCTCCTTCTTAAAGTTAAACAAAATTATTTCTAGAGGGG | 17 |
| 380_KSR205-for | TAATGAGATCCGGCTGCTAACAAAGCC | 18 |
| 381_KSR205-rev | GCTACCACCACCACCCACACCAT | 19 |
| 382_KSR206-for | CACCCGTCCGGATAATGGTGTGGGTGGTGGTGGTAGCGAAATTGGCACCGGT TTTC | 20 |
| 383_KSR206-rev | CTTTCGGGCTTTGTTAGCAGCCGGATCTCATTAACCGCCGCTAATTTCCAGG GTGC | 21 |
| KSR_168 | CGTGATTTTGCAGGAGACGGGTTAGTTAC | 22 |
| KSR_169 | GTCTCCTGCAAAATCACGTACAAATTTAAGACCTTG | 23 |
| MS1 | GGGGACAAGTTTGTACAAAAAAGCAGGCTCTGAAAACCTGTATTTTCAGTGC TCAGTTTTTGTTTCAGGTG | 24 |
| MS2 | GGGGACCACTTTGTACAAGAAAGCTGGGTGTTATATTCTGCCCTCAAATTTT AAAA | 25 |
| TP17 | GCTACCACCACCACCCACACCATTATC | 26 |
| TP18 | TAATGAGATCCGGCTGCTAACAAAGC | 27 |
| TP19 | CCGGATAATGGTGTGGGTGGTGGTGGTAGCTCAGTTTTTGTTTCAGGTGCTA ACG | 28 |
| TP20 | TCGGGCTTTGTTAGCAGCCGGATCTCATTATATTCTGCCCTCAAATTTTAAA AT | 29 |
| TP25 | ATGATGGTGGTGATGATGCATGTATATCTCCTTCTTAAAGTTAAACAAAATT ATTTC | 30 |
| TP26 | CATCATCACCACCATCATGCACATATTGTTATGGTTGATGCCTATAAACCG | 31 |
| TP27 | CTTTAAGAAGGAATATCACAAGTTTGTACTAAGAAGGAGATATACATATG | 32 |
| TP28 | GGTGGTGGTGGTAGCGCCCATATTGTTATGGTGGATGCATATAAAC | 33 |
| TP35 | AGTTTGTACTAAGAAGGAGATATACATATGACAATTAAAGAAATGCCTCAGC CAAAAAC | 34 |
| TP36 | CATAACAATATGGGCGCTACCACCACCACCCCCAGCCCACACGTCTTTTGC | 35 |
| SG3 | GGGCATCATCACCACCATCATTAATGAGATCCGGCTGCTAACAAAGCCCG | 36 |
| SG4 | TTAATGATGGTGGTGATGATGCCCAGCCCACACGTCTTTTGC | 37 |
| SG7 | TATAAACCGACCAAAGGTGGTGGTGGCAGCACAATTAAAGAAATGCCTCAGC CAAAAAC | 38 |
| SG8 | CTTTCGGGCTTTGTTAGCAGCCGGATCTTACCCAGCCCACACGTCTTTTGC | 39 |

Das eGFP mit einem N-terminal fusionierten G₄S-Linker (SEQ ID NO: 1) und einer SC-Sequenz (SC-eGFP-His) wurde als Codon-optimiertes Genfragment mit überlappenden Sequenzen zu dem pET22b-Vektorrückgrat erworben (Geneart, Deutschland) und dieses Vektorrückgrat unter Verwendung der Primer 41_KSR23_for und 42_KSR23_rev linearisiert. Nach dem Zusammenbau wurde das resultierende pET_SC-eGFP-His-Plasmid als Template in einer PCR-Reaktion mit den Primern TP7 und TP8 verwendet, um die SC DNA-Sequenz zu entfernen und durch die SpyTag (ST)-Sequenz zu ersetzen, was zu dem Plasmid pET_ST-eGFP-His führte. Um den Expressionsvektor für SC zu erzeugen, wurde ein synthetisches, Codon-optimiertes DNA-Fragment von SC, enthaltend eine N-terminale Hexahistidin-Markierung, eine TEV-Stelle und ein Stopcodon mit zusätzlichen attB-Stellen, erworben (Geneart) und mit dem Vektor pDONR221 unter Verwendung der Gateway BP-Reaktion rekombiniert. Das erhaltene Plasmid pENTR221_His-SC wurde dann einer LR-Reaktion mit dem Plasmid pDESTn1 unterzogen, um das finale Expressionsplasmid pEXPn1_His-SC zu erhalten. Um den Expressionsvektor für LbADH zu erzeugen, wurde ein synthetisches, Codon-optimiertes DNA-Fragment von LbADH, flankiert von zusätzlichen attB-Stellen, erworben (Geneart) und mit dem Vektor pDONR221 in einer Gateway BP-Reaktion rekombiniert. Das erhaltene Plasmid pENTR221_LbADH-His wurde dann einer LR-Reaktion mit dem Plasmid pDESTn3 unterzogen, um das finale Expressionsplasmid pEXPn3_His-LbADH zu erhalten. Die genomische DNA kommerziell erhältlicher Bäckerhefe *(Saccharomyces cerevisiae* YJM193) wurde mittels InstaGeneTM Matrix (Biorad) isoliert und das Gre2p-Gen yol151w mit den Primern MS1 und MS2 via PCR amplifiziert und dabei mit attB-Stellen modifiziert. Die weitere Klonierung erfolgte analog zu der der LbADH. Um den Expressionsvektor für SBP-mKate zu erzeugen, wurde ein Codon-optimiertes DNA-Fragment, enthaltend eine Sequenz enthaltend die Ribosomen-Bindestelle (RBS), SBP und das mKate Protein, flankiert von den attB-Stellen attB1 und attB2, erworben (Geneart). Dieses DNA-Fragment wurde mit pDONR221 in einer Gateway BP-Reaktion rekombiniert, um den Eintrittsvektor pENTR221_SBP-mKate zu erhalten. Der Eintrittsvektor wurde dann weiter mit pDESTn8 umgesetzt, um pEXPn8_SBP-mKate-SNAP-His zu erhalten.

### Klonierung der LPP-ompA-Konstrukte.

Das Plasmid pET_Lpp-ompA-ST wurde aus einem DNA-Fragment, enthaltend eine Codon-optimierte Version von Lpp-ompA, einen C-terminal fusionierten G₄S-Linker (SEQ ID NO: 1), die ST-Sequenz und das linearisierte Vektorrückgrat pET22b, konstruiert. Letzteres wurde durch PCR unter Verwendung der Primer 124_KSR65_for und 125_KSR65_rev erhalten. Das Plasmid pET_Lpp-ompA-HOB wurde durch Zusammenbau der HOB-Sequenz aus pET-eCFP-HOB, welches mit den Primern 380_KSR205-for und 381_KSR205-rev amplifiziert wurde, und des Vektorrückgrats pET_Lpp-ompA-ST, welches unter Verwendung der Primer 382_KSR206-for und 383_KSR206-rev linearisiert wurde, erzeugt. Das Plasmid pET_Lpp-ompA-SBP wurde durch PCR mit den Primern TP1 und TP2 und pET_Lpp-ompA-HOB als Template erhalten. Die Überhänge enthielten die SBP-Markierung. Das Plasmid pET_Lpp-ompA-SC wurde unter Verwendung des pET_Lpp-ompA-HOB-Vektorrückgrats, welches mit den Primern TP3 und TP4 linearisiert wurde, und eines SC-Inserts, welches von pEXPn1_His-SC mit den Primern TP5 und TP6 amplifiziert wurde, zusammengebaut.

In einer anfänglichen Phase des vorliegenden Projekts wurden alle Proteinkonstrukte von dem pET22b-Plasmid exprimiert. Aufgrund der Durchlässigkeit (Basal-Transkription und -Expression) des T7-Promotors waren dabei das Zellwachstum und die Effizienz der Oberflächenexpression selbst ohne IPTG-Induktion signifikant eingeschränkt. Diese Beobachtung stimmt mit früheren Studien überein, die berichten, dass die Expression von ompA-Fusionsproteinen von einem Plasmid mit hoher Kopienzahl, welches einen durchlässigen Promotor verwendet (beispielsweise pET), zu langsamem Wachstum, erheblichem Ausströmen von periplasmatischen Enzymen oder sogar Zelllyse führt. Daher wurden alle Inserts in das pTF16-Rückgrat (kommerziell erhältlich in dem Chaperone Plasmid Set von Takara Bio, Deutschland) subkloniert und unter Verwendung des Arabinose-Promotors exprimiert. Die Primer TP11 und TP12 wurden verwendet, um die RBS-Lpp-OmpA-TAG enthaltenden Inserts unter Verwendung von pET_Lpp-ompA-ST, pET_LppompA-HOB, pET Lpp-ompA-SBP oder pET Lpp-ompA-SC als jeweilige Templates zu erzeugen. Der Zusammenbau dieser Inserts mit dem pTF16-Rückgrat, welches unter Verwendung der Primer TP13 und TP14 linearisiert wurde, führte zu den entsprechenden pTF16_Lpp-ompA-Konstrukten.

### Klonierung der P450 BM3-Konstrukte.

Für die Erstellung von pTF16_Lpp_ompA-P450 BM3(A74G F87V)-His wurde das Vektorrückgrat durch PCR mit den Primern TP17 und TP18 auf das Template pTF16_ompA-SBP linearisiert. Das Insert wurde mit TP19 und TP20 auf das Plasmid pET-DESTn1_BM3(A74G F87A) amplifiziert und mit dem Vektorrückgrat rekombiniert. In das erstellte Plasmid wurde durch PCR mit SG3 und SG4 ein C-terminales His-Tag angehängt und mit den Primern KSR168 und KSR169 in einer ortsgerichteten Mutagenese das F87A zu einem F87V mutiert.

Für das N-terminale Markieren der Proteine mit ST und His wurde ein Vektorrückgrat mit den Primern TP25 und TP26 auf das Template pET_ST-eGFP amplifiziert und dabei ein His-Tag eingeführt. Das Vektorrückgrat wurde anschließend mit dem Insert, welches mittels PCR mit den Primern SG 7 und SG 8 und dem Template pTF16_Lpp_ompA-P450 BM3(A74G F87V)-His erstellt wurde, rekombiniert und so das Plasmid pET_His-ST-P450BM3(A74G F87V) erhalten.

Zur Konstruktion des Plasmids für das C-terminale Markieren der Proteine mit ST und His wurde mittels einer PCR mit dem Template pET-DESTn23_Gre2p-STHis und den Primern TP 27 und TP28 ein lineares Vektorrückgrat generiert. Danach folgte die Rekombination mit dem Insert, welches durch PCR mit pET-DESTn1_BM3(A74G F87A) und den Primern TP35 und TP36 amplifiziert wurde. So konnte das finale Plasmid pET_P450BM3(A74G F87A)-ST-His, bei dem das F87A mit den Primern KSR168 und KSR169 zu einem F87V mutiert wurde, erstellt werden.

### Expression und Aufreinigung der Proteine.

Für die heterologe Proteinexpression wurden E. *coli* BL21 (DE3) mit den entsprechenden Expressionsvektoren transformiert und bei 37°C in 2 L LB-Medium in einer Schüttelflasche kultiviert, bis die OD600 einen Wert von 0,6 erreicht hat. Die Temperatur wurde dann auf 25°C gesenkt und IPTG zu einer finalen Konzentration von 0,1 mM für zusätzliche 16 Stunden zugegeben. Die Zellen wurden durch Zentrifugation (10000 × g, 10 Min) geerntet und in 30 mL Puffer A (50 mM NaH₂PO₄, 300 mM NaCl, 10 mM Imidazol, pH 8,0) resuspendiert. Nach Aufspaltung durch Ultraschallbehandlung wurde das Zelllysat durch Zentrifugation (45000 × g, 1 h) erhalten, durch eine 0,45 µm Durapore PVDF-Membran (Steriflip, Millipore) gefiltert und auf eine HisTrap FF (5 mL) Ni-NTA-Säule (GE Healthcare, Deutschland) geladen, die an einem Äkta Pure Flüssigchromatographiesystem angebracht war. Die Säule wurde mit 50 mL Puffer A gewaschen und die 6 × His-markierten Proteine mit 100% Puffer B (50 mM NaH₂PO₄, 300 mM NaCl, 500 mM Imidazol, pH 8,0) eluiert. Nachfolgend wurde der Puffer mittels Vivaspin 10000 MWCO (GE Healthcare) gegen 100 mM KPᵢ, pH 7,5 ausgetauscht. Für die Aufreinigung des SBP-markierten Proteine wurde eine 5 mL Strep-Tactin^{®} Superflow^{®} Kartusche von iba-lifescience (Deutschland) gemäß der Anweisungen des Herstellers verwendet.

Um die rekombinanten aufgereinigten Proteine zu charakterisieren, wurden typischerweise Proben durch 16% Gelelektrophorese mit herkömmlichen diskontinuierlichen SDS-Polyacrylamid-Laemmli-Midi-Gelen analysiert. Die Banden wurden durch Coomassie-Färbung sichtbar gemacht und mit der Page Ruler^{™} Prestained Protein Ladder (Thermo Scientific) verglichen. Die Konzentrationen wurden mittels UV-Vis-Spektroskopie unter Verwendung der theoretischen molaren Extinktionskoeffizienten bei 280 nm, die von der Software Geneious, Version 8.0.5 berechnet wurden, bestimmt. *Synthese der CH-modifizierten Oligonukleotide.*

Die Synthese von Chlorhexyl (CH)-modifizierten Oligonukleotiden wurde wie im Stand der Technik bekannt durchgeführt. Kurz dargestellt wurde das 3'-Alkylamino-modifizierte Oligonukleotid Cy5-F5-AmC7 (GGT CCG GTC ATA AAG CGA TAA G; SEQ ID NO: 40) von Sigma erworben und mittels DNA Clean & Concentrator (Zymo Research) entsalzt. 50 µL von 100-400 µM Oligonukleotid in 0,5 × PBS, pH 8,6 (1,75 mM NaH₂PO₄, 4 mM Na₂HPO₄, 75 mM NaCl) wurden mit HaloTag-Succimidylester (O4)-Ligand (Promega, 50 pL von 10 mM, gelöst in trockenem DMSO) für 18 h bei 25 °C umgesetzt. Die Reaktionsmischung wurde mittels HPLC aufgereinigt. Reversed-Phase-HPLC-Chromatographie der CH-modifizierten Oligonukleotide wurde auf einem Agilent Technologies 1200-Series System unter Verwendung einer Zorbax Eclipse XDB-C18-Säule (4,6 × 150 mm, Agilent) durchgeführt. 0,1 M Ammoniumacetat (Lösungsmittel A) und Acetonitril (Lösungsmittel B) wurden als Elutionsmittel verwendet. Ein linearer Gradient 0-100% Lösungsmittel B über 40 Minuten mit einer Flussrate von 1 mL/Min wurde angewandt. Der Signalnachweis wurde mittels UV-Detektion bei 260 nm und 280 nm durchgeführt. Die Proben wurden dann durch Eindampfen konzentriert und mit einem DNA Clean & Concentrator entsalzt. Die Konzentration wurde mittels UV-Vis-Spektroskopie bestimmt und die Reinheit zusätzlich mit 25% Harnstoffgelen bestätigt.

### Funktionalisierung der magnetischen Mikropartikel.

Die für die Immobilisierung von SBP-markierten Zellen verwendeten magnetischen Mikropartikel waren Dynabeads C1 Streptavidin (MB-STV) von Thermo Fisher Scientific. Ähnliche Ergebnisse wurden mit Dynabeads M-270 Streptavidin erhalten (Fig. 8). Magnetische Partikel, die einen Chlorhexylliganden an ihrer Oberfläche enthalten (MB-CH), wurden aus Dynabeads C1 Streptavidin Kügelchen durch Inkubation mit Biotin-Chlorhexyl-Konjugat erhalten, gelöst in 100 mM KPᵢ, pH 7,5 bei einer finalen Konzentration von 2 nmol Biotin-Chlorhexyl pro mg Kügelchen. Die Reaktionsmischung wurde für mindestens 1 h bei RT und 1000 UpM inkubiert und die Kügelchen nachfolgend mit 100 mM KPᵢ, pH 7,5 gewaschen. SC auf ihrer Oberfläche enthaltende magnetische Partikel (MB-SC) wurden, gemäß der Anleitung des Herstellers, durch kovalente Immobilisierung des SC Proteins (Fig. 2) auf Dynabeads Epoxy (Thermo Fisher Scientific) über dessen Lysinreste erzeugt.

Die Zugänglichkeit der Liganden auf den funktionalisierten Partikeln wurde durch Inkubation mit den entsprechenden, Fluoreszenz-markierten Interaktionspartnern getestet. In einem typischen kompetitiven Bindungsassay wurden 4 nmol Halo-eGFP und SBP-mKate pro mg von jeweils MB-STV oder MB-CH in 100 mM KPᵢ, pH 7,5 für 1 h bei RT inkubiert. Die Kügelchen wurden mit einem Magnet gesammelt und mit 100 mM KPᵢ, pH 7,5 gewaschen und mittels Fluoreszenzmikroskopie analysiert. Der Funktionalitätstest von MB-SC wurde auf gleiche Weise durchgeführt, wobei eine höhere Konzentration von 20 nmol/mg von SC-eGFP oder ST-eGFP verwendet wurde.

### Fluoreszenzmikroskopie.

Die mikroskopischen Analysen wurden manuell mit einem Zeiss Axiovert 200M Fluoreszenzmikroskop und der Software Axio Vision 4.7 durchgeführt. 20 µL der Proben wurden zwischen zwei Objektträger pipettiert und Fluoreszenzaufnahmen von verschiedenen Stellen der Objektträger in vier verschiedenen Fluoreszenzkanälen aufgenommen. Das Cy5-Signal wurde mit einem Cy5-Filtersatz (violetter Kanal) analysiert, das eGFP- oder YFP-Signal mit einem FITC-Filtersatz (grüner Kanal), mKate und Cy3 mit eine Rhodamin-Filtersatz (roter Kanal) und DAPI mit einem DAPI-Filtersatz (blauer Kanal).

### Erzeugung und Charakterisierung rekombinanter E. coli Zellen, die orthogonale Markierungseinheiten auf ihrer Oberfläche präsentieren.

Die Plasmide pTF16_Lpp-ompA-SBP, pTF16_Lpp-ompA-HOB, pTF16_Lpp-ompA-ST und pTF16_Lpp-ompA-SC wurden mittels Elektroporation in E. *coli* BL21 (DE3) transformiert. Die frisch transformierten E. *coli* Zellen, welche die verschiedenen Proteinmarkierungs-Plasmide enthielten, wurden über Nacht bei 37 °C auf LB/Agar-Platten, enthaltend 30 µg/µL Chloramphenicol, selektiert. Flüssigkulturen (20 mL LB-Medium, enthaltend Chloramphenicol) wurden direkt von den Übernachtkulturen auf LB/Agar-Platten in 50 mL Schüttelflaschen bei 37 °C und 180 UpM angelegt. Wenn die OD600 einen Wert von etwa 0,6 erreicht hatte, wurde die Expression der OmpA-Fusionsproteine durch Zugabe von Arabinose bei einer Endkonzentration von 1mM induziert. Die Zellen wurden bei 30 °C für 8 bis 10 Stunden wachsen gelassen, durch kurze Zentrifugation gesammelt und in 100 mM KPᵢ, pH 7,5 zu einer Dichte von 0,8 OD600/mL resuspendiert. 500 µL Aliquots dieser Zellsuspension wurden für 1 h bei 1000 UpM bei Raumtemperatur (RT) mit 100 pmol von entweder ST-eGFP, SC-eGFP, STV-Cy3 oder Cy5-F5-CH inkubiert, um auf spezifische Bindung zu testen. Die Zellen wurden dreimal gewaschen, indem die Zellen durch Zentrifugation gesammelt und in 1 mL 100 mM KPᵢ, pH 7,5 resuspendiert wurden. Nach dem letzten Waschschritt wurden die Zellen in 100 µL 100 mM KPᵢ, pH 7,5 resuspendiert und in 96-well Black MaxiSorp-Platten (Thermo Scientific) transferiert. Die Fluoreszenz wurde mit einem Synergy^{™} H1m Monochromator-basierten Multi-Mode Mikroplattenleser (BioTek) unter Verwendung der folgenden Filtersätze bestimmt: GFP (Exc 490nm, Em 510nm), Cy3 (Exc 550nm, Em 570nm) und Cy5 (Exc 650nm, Em 670nm). Die relativen Fluoreszenzwerte wurden auf den höchsten Wert innerhalb jedes Datensatzes normalisiert. Die gleichen Proben wurden nachfolgend durch Zugabe von 10 µL einer 1:1000-Lösung einer DAPI-Färbungs-Stammlösung (14,3 mM) in PBS mit DAPI gefärbt und für 20 Min inkubiert. Die DAPI-gefärbten Zellen wurden dann mittels Fluoreszenzmikroskopie analysiert.

### YFP@E.coli-markierte Varianten.

YFP@E.coli-markierte Varianten wurden durch Transformation von E. *coli* BL21 (DE3) mit dem eYFP-Plasmid und einem der pTF16 Lpp-ompA-Markierungsplasmide (pTF16_Lpp-ompA-SBP, pTF16_Lpp-ompA-HOB, pTF16_Lpp-ompA-ST oder pTFl6_Lpp-ompA-SC) mittels Elektroporation erzeugt. Die frisch transformierten YFP@E. coli-Markierungszellen wurden über Nacht bei 37 °C auf LB/Agar-Platten, enthaltend 100 µg/µL Ampicillin und 30 µg/µL Chloramphenicol, selektiert. Klone wurden ausgewählt und von den LB/Agar-Platten in Flüssigkulturen überführt und über Nacht bei 37 °C in 5 mL LB, enthaltend Ampicillin und Chloramphenicol, inkubiert. Am nächsten Tag wurden 20 mL LB, enthaltend Ampicillin und Chloramphenicol, in 50 mL Schüttelflaschen mit bis zu 5 % der Übernachtkulturen inokuliert. Nach zwei Stunden Inkubation bei 37 °C, 180 UpM wurde eine OD600 von etwa 0,6 erreicht und die Proteinproduktion durch Zugabe von Arabinose (10 µM Endkonzentration) und IPTG (100 µM Endkonzentration) induziert. Die Zellen wurden bei 30 °C für 8 bis 10 Stunden inkubiert, die OD600 bestimmt und die Zellen durch kurze Zentrifugation gesammelt.

Die Zellen wurden dann in 100 mM KPᵢ, 500 mM NaCl, pH 6,0 bis zu einer theoretischen Dichte von 150 OD600 resuspendiert. 20 µL dieser Zellsuspension wurden dann mit 100 µg der Liganden-enthaltenden Kügelchen in einem Gesamtvolumen von 1 mL 100 mM KPᵢ, 500 mM NaCl, pH 6,0 für eine Stunde bei 30 °C mit Überkopfmischen der Reaktionsröhrchen inkubiert. Nachfolgend wurden die Zellen dreimal gewaschen, indem die Zellen durch Zentrifugation gesammelt und in 1 mL 100 mM KPᵢ, 500 mM NaCl, pH 6,0 resuspendiert wurden. Nach dem letzten Waschschritt wurden die Zellen in 20 µL 100 mM KPᵢ, 500 mM NaCl, pH 6,0 resuspendiert und in einem Fluoreszenzmikroskop bei Einsatz eines FITC-Filtersatzes analysiert.

### KRED@E.coli-markierte Varianten.

KRED@E.coli-markierte Varianten wurden durch Transformation von E. *coli* BL21 (DE3) mit Kombinationen der die Ketoreduktasen LbADH oder Gre2p enthaltenden Plasmide und einem der pTF16 Lpp-ompA-Markierungsplasmide (pTF16_Lpp-ompA-SBP, pTF16_Lpp-ompA-HOB, pTF16_Lpp-ompA-ST oder pTFl6_Lpp-ompA-SC) mittels Elektroporation erzeugt. Die frisch transformierten KRED@E. coli-Markierungszellen wurden über Nacht bei 37 °C auf LB/Agar-Platten, enthaltend 100 µg/µL Ampicillin und 30 µg/µL Chloramphenicol, selektiert. Die Kultivierung der LbADH@E.coli-SBP, LbADH@E.coli-HOB, LbADH@E.coli-ST, Gre2p@E.coli-SBP, Gre2p@E.coli-HOB und LbADH@E.coli-ST wurde direkt durch Überführen der auf LB/Agar-Platten gewachsenen Übernachkulturen in Flüssigkulturen (20 mL LB, enthaltend Ampicillin und Chloramphenicol) in 50 mL Schüttelflaschen bei 37 °C und Schütteln bei 180 UpM gestartet. Nachdem eine OD600 von etwa 0,6 erreicht wurde, wurde die Proteinproduktion durch Zugabe von Arabinose (10 µM Endkonzentration) und IPTG (100 µM Endkonzentration) induziert. Die Zellen wurden bei 30 °C für 8 bis 10 Stunden inkubiert, die OD600 bestimmt und die Zellen durch kurze Zentrifugation gesammelt. Die Zellen wurden dann in 100 mM KPᵢ, 500 mM NaCl, pH 6,0 bis zu einer theoretischen Dichte von 150 OD600 resuspendiert. 10 µL je individueller Kombination von Oberflächenmodifikation und KRED-Gehalt exprimierendem Zelltyp wurden dann mit 100 µg der Kügelchen in einem Gesamtvolumen von 1 mL 100 mM KPᵢ, 500 mM NaCl, pH 6,0 für eine Stunde bei 30 °C mit Überkopfmischen der Reaktionsröhrchen inkubiert. Nachfolgend wurden die Zellen durch Sammeln und Resuspendierung wie vorstehend beschrieben gewaschen und schließlich in 200 µL 37 °C LB, enthaltend Ampicillin und Chloramphenicol, resuspendiert, in eine neues 1,5 mL Eppendorf-Röhrchen überführt und nach Zugabe von weiteren 1 mL 37 °C LB, enthaltend Ampicillin und Chloramphenicol, für 3 Stunden bei 37 °C inkubiert. Nachfolgend wurden 100 µL dieser Zellsuspension mit 500 µL LB, enthaltend Ampicillin und Chloramphenicol, zusammen mit 100 µM IPTG und 5 mM des 5-Nitronona-2,8-dions (NDK) 1 bei 30 °C für 8 bis 10 Stunden inkubiert. Um die organischen Verbindungen zu extrahieren wurden Aliquots von 100 µL genommen und kräftig mit 100 µL Ethylacetat gemischt. 50 µL der organischen Phase wurden entfernt, in einem Eppendorf Concentrator plus abgedampft und mittels chiraler HPLC analysiert.

### Chirale HPLC-Analyse.

Die Synthese und Charakterisierung von NDK 1, sowie die Analyse der biokatalytischen Reaktionsprodukte mittels chiraler HPLC, wurden wie im Stand der Technik bekannt durchgeführt. Kurz dargestellt wurden die getrockneten Ethylacetatextraktionen aus den rohen Reaktionsmischungen (vorstehend beschrieben) in 100 µL der mobilen Phase (90% n-Heptan, 10% 2-Propanol) resuspendiert und 10 µL der Lösung in das HPCL-Gerät (Agilent 1260 Serie HPLC, ausgestattet mit einem Diodenarraydetektor (210 nm) auf einer chiralen Lux 3µ Cellulose-1 (150 × 2,00 mm) Säule (Phenomenex)) injiziert. Identische Laufbedingungen wurden für die Analyse der Hydroxyketone (2), wie im Stand der Technik für Verfahren A bekannt, verwendet (Chromatographie-Lösungsmittel 90% n-Heptan/ 10% 2-Propanol, 10 Min isokratisch, Säulenofentemperatur von 10 °C und eine Flussrate von 0,5 mL/Min). Beispielhafte HPLC-Diagramme sind in Figur 3 gezeigt.

### Bestimmung der Aktivität ganzer Zellen.

KRED@E.coli-markierte Bakterien wurden über Nacht bei 37 °C in 5 mL LB, enthaltend 100 µg/mL Ampicillin und 30 µg/mL Chloramphenicol, inkubiert. Am nächsten Tag wurden 20 mL LB, enthaltend Ampicillin und Chloramphenicol, in 50 mL Schüttelflaschen zu bis zu 5 % mit den Übernachkulturen inokuliert. Nach zwei Stunden Inkubation bei 37 °C mit Schütteln bei 180 UpM und einer OD600 von etwa 0,6 wurde die Proteinproduktion durch Zugabe von 1 mM Arabinose und 100 µM IPTG (Endkonzentrationen) induziert. Die Zellen wurden bei 30 °C für weitere vier Stunden inkubiert, die OD600 bestimmt und, nach Sammeln der Zellen durch eine kurze Zentrifugation, die Zellen in 200 µL LB (enthaltend Ampicillin, Chloramphenicol, 1 mM IPTG und 10 mM des Substrats NDK 1) zu einer Dichte von 2,5 OD600/mL resuspendiert. Die Reaktionsmischung wurde für zwei Stunden bei 30 °C und 100 UpM Schütteln inkubiert. Nachfolgend wurden 50 µL Probenaliquots kräftig mit 100 µL Ethylacetat gemischt, um die organischen Verbindungen aus der wässrigen Phase zu extrahieren. 50 µL der Proben der organischen Phase wurden entfernt, abgedampft und durch chirale HPLC, wie vorstehend beschrieben, analysiert.

### Oberflächenpräsentation von Proteinen.

P450 BM3(A74G F87V)-ST@E.coli-SC wurde durch Transformation von E. *coli* BL21 (DE3) mit dem die ST-getaggte Oxidoreduktase P450 BM3(A74G F87V) enthaltenden pET22b basierten Plasmid und pTF16_Lpp-ompA-SC mittels Elektroporation erzeugt. Die frisch transformierten P450 BM3(A74G F87V)-ST@E.coli-SC wurden über Nacht bei 37 °C auf LB/Agar-Platten, enthaltend 100 µg/µL Ampicillin und 30 µg/µL Chloramphenicol, selektiert und anschließend eine 5 ml LB Vorkultur, mit denselben Antibiotika, mittels eines Klones beimpft. Am Folgetag wurde 1 mL der Übernachtkulturen in Flüssigkulturen (20 mL LB, enthaltend Ampicillin und Chloramphenicol) in 50 mL Schüttelflaschen überimpft und bei 37 °C und Schütteln bei 180 UpM inkubiert. Nachdem eine OD600 von etwa 0,6 erreicht wurde, wurde die Proteinproduktion durch Zugabe von Arabinose (1 mM Endkonzentration) und IPTG (1 mM Endkonzentration) induziert und für weitere 4 h bei 25°C und 180 UpM inkubiert. Die Zellen wurden anschließend durch kurze Zentrifugation gesammelt und in 100 mM Tris-Base, pH 8,1 bis zu einer theoretischen Dichte von OD600 von 0,5 resuspendiert. Die kinetische Messung der enzymatischen Umsetzung des fluorogenen BM3 Substrates (4) erfolgte in einem Mikrotiterplattenleser Synergy H1 in einem Gesamtvolumen von 200 µl 100 mM Tris-Base, pH 8,1 mit P450 BM3(A74G F87V)-ST *@E.coli-SC* OD600 von 0,025, 1 mM NADPH und 100 µM Substrat HTC **4** (12-(4-trifluoromethylcoumarin-7-yloxy)dodecanoate) für 60 Minuten. Die Produktbildung von **6** wurde bei einer Anregung von 420 nm und einer Emission von 500 nm verfolgt und mit Kalibrierlösungen verglichen.

### Beispiel 1:

Zur experimentellen Überprüfung des der vorliegenden Erfindung zugrunde liegenden Konzepts wurden drei verschiedene orthogonale Markierungssysteme ausgewählt, welche soweit noch nicht zur spezifischen Immobilisierung von ganzen, lebenden Zellen verwendet wurden. Das aus 39 Aminosäuren bestehende SBP bindet mit hoher Affinität an Streptavidin und wird in der chromatographischen Aufreinigung von Proteinen eingesetzt. Das SpyTag/SpyCatcher-System besteht aus dem SpyCatcher-Protein (113 Aminosäuren), welches eine kovalente Isopeptidbindung zwischen einem seiner Lysinreste und einem Asparaginrest des SpyTag-Peptids (13 Aminosäuren) ausbilden kann. Das selbst-markierende HOB-Protein (293 Aminosäuren) bildet eine kovalente Bindung mit kleinen Chlorhexanliganden aus, ähnlich dem Halo-Tag-Protein, welches in zellbiologischen Bildgebungsverfahren verwendet wird. HOB wurde gentechnisch optimiert, um Chlorhexanliganden auf DNA-Oligonukleotiden und -Nanostrukturen mit signifikant höherer Effizienz zu binden.

Plasmide, die für Fusionsproteine aus dem bakteriellen Membranprotein Lpp-ompA und daran über einen GGGGS-Linker (SEQ ID NO: 1) gekoppelte SBP-, ST-, SC- oder HOB-Markierungen kodieren, wurden in das pTF16-Rückgrat kloniert, welches eine Chloramphenicolresistenz und einen p15A-Ori trägt und in dem die Proteinexpression streng über einen Arabinose-abhängigen Promotor kontrolliert wird. Die Transformation von E. *coli* BL21 (DE3) mit den hergestellten Plasmiden führte zur Bildung rekombinanter Bakterien, die entweder eine SBP-, ST-, SC- oder HOB-Markierung an ihrer Zelloberfläche präsentieren (im Folgenden jeweils als E. coli-SBP, E. *coli-STP, E. coli-*ST oder E. *coli-HOB* bezeichnet).

### Beispiel 2:

Zunächst wurde die Zugänglichkeit und Bindungsselektivität der präsentierten Markierungen untersucht. Dazu wurden Fluoreszenz-markierte Sonden (Cy3-markiertes STV, SC-eGFP und ST-eGFP Fusionsprotein, oder ein mit Cy5 markiertes, mit Chlorhexan derivatisiertes 22-mer Oligonukleotid (Cy5-F5-CH)) mit entsprechenden Zellsuspensionen inkubiert. Nach Inkubation für eine Stunde wurden die Zellen abzentrifugiert und der Überstand entfernt. Nach Waschen der Zellen wurden diese fluoreszenzmikroskopisch analysiert (Fig. 5A) und entsprechende quantitative Daten mit einem fluorimetrischen Mikroplattenleser erhoben (Fig. 5B). Die Ergebnisse zeigen klar, dass alle vier Markierungen auf den Zelloberflächen zugänglich sind und ihre Interaktionspartner in hoch-spezifischer Weise binden.

### Beispiel 3:

Nachfolgend wurde untersucht, ob die an den Zelloberflächen präsentierten Markierungen für die Immobilisierung der Zellen auf magnetischen Mikropartikeln verwendet werden können. Dazu wurden mit STV, Chlorhexanliganden oder SC-Protein beschichtete Mikropartikel (nachfolgend jeweils als MB-STV, MB-CH und MB-SC bezeichnet) verwendet. MB-STV war kommerziell erhältlich, MB-CH wurden aus MB-STV und Biotin-Chlorhexan und MB-SC durch kovalente Immobilisierung von gereinigtem SC-Protein auf Amino-reaktive, Epoxid-beschichtete Mikropartikel hergestellt. Die Funktionalität der drei verschiedenen Mikropartikel wurde durch kompetitive Bindungsstudien unter Verwendung aufgereinigter, markierter fluoreszierender Proteine bestätigt (SBP-, Halo- und ST-markiertes eGFP und mKate; Fig. 6). Die Ergebnisse zeigen klar, dass die Mikropartikel in der Lage sind, die komplementären Sonden spezifisch zu immobilisieren.

### Beispiel 4:

Nachfolgend wurden Markierungen präsentierende E. coli-Stämme hergestellt, die weitere Plasmide für die Überexpression heterologer, funktioneller Proteine in ihrem Cytosol enthielten. Diese Plasmide basierten auf dem pET-Plasmidrückgrat, so dass diese vollständig orthogonal zu den pTF16-basierten Immobilisierungsplasmiden waren, da sie eine Ampicillinresistenz, einen colE1-Ori und einen T7-Promotor, welcher selektiv mit IPTG induziert werden kann, tragen.

Als erstes Modell für ein funktionelles Protein wurde YFP *(yellow fluorescent protein)* untersucht. Dazu wurden die Stämme YFP@E.coli-SBP, YFP@E.coli-ST und YFP@E.coli-HOB erzeugt. Bindungsstudien wurden durchgeführt, indem die Zellen entweder mit MB-STV, MB-SC oder MB-CH Mikropartikeln inkubiert wurden. Nach der Inkubation wurden die Mikropartikel durch magnetische Trennung isoliert und mittels Fluoreszenzmikroskopie analysiert (Fig. 7). Die entsprechenden Bilder zeigen klar, dass die drei verschiedenen E. coli-Stämme nur an die Mikropartikel binden, welche die komplementären Bindungseinheiten enthalten. Des Weiteren führt die polyvalente Bindung, da sowohl die Mikropartikel als auch die Zellen eine Vielzahl von Kopien der Interaktionspartner präsentieren, zur Bildung von Aggregaten aus Zellen und Mikropartikeln (Fig. 8).

### Beispiel 5:

Da immobilisierte bakterielle Zellen als wertvolle Quelle für Biokatalysatoren in der Herstellung chiraler Verbindungen in Betracht kommen, wurde die Nützlichkeit der vorliegenden Erfindung für Anwendungen in der Ganzzellbiokatalyse untersucht. Dazu wurden Markierungen präsentierende E. coli-Stämme erzeugt, welche ein weiteres Plasmid für die Überexpression stereoselektiver Enzyme enthielten. In früheren Studien wurde eine Reihe von hoch-stereoselektiven Ketoreduktasen (KREDs) für die Reduktion des prochiralen Cs-symmetrischen Nitrodiketon (NDK) 1 (Fig. 9) identifiziert. Abhängig von der Selektivität des Enzyms kann das Substrat 1 an entweder jeweils einer oder an beiden der zwei Carbonylfunktionen reduziert werden und es können alle möglichen isomeren Hydroxyketon- und Diolprodukte getrennt und mittels chiraler HPLC-Analyse analysiert werden. Daher wurde die Transformation von **1** als ideales Modellsystem gesehen, um die Aktivität und die Bindungsspezifität der erfindungsgemäßen selbst-immobilisierenden Biokatalysatoren zu untersuchen.

Es wurden *E. coli*-Stämme erzeugt, in denen die an der Zelloberfläche präsentierten SBP-, HOB- oder ST-Markierungen entweder mit der (R)-selektiven Alkoholdehydrogenase aus *Lactobacillus brevis* ATCC 14869 (LbADH) oder der (S)-selektiven Alkoholdehydrogenase aus *Saccharomyces cerevisiae* YJM193 (Gre2p) kombiniert wurden (Stämme LbADH@*E.coli*-SBP, LbADH@*E.coli*-HOB, LbADH@*E.coli*-ST, Gre2p@*E.coli*-SBP, Gre2p@*E.coli*-HOB und Gre2p@*E.coli*-ST; Tabelle 2). Um zu testen, ob die Präsentation der Markierungen die biokatalytischen Eigenschaften beeinflusst, wurde zunächst die enzymatische Aktivität des KRED-Inhalts ganzer Zellen mittels chiraler HPLC bestimmt (Zeilen 1 bis 6 in Tabelle 2). Im Falle von LbADH exprimierenden Zellen wurde NDK **1** mit einer Diastereoselektivität von >99% in die (R)-konfigurierten Hydroxyketone **2c/2d** und das Diol **3d** umgewandelt, wie mittels chiraler HPLC-Analyse bestimmt (Zeilen 1 bis 3 in Tabelle 2). Im Gegensatz dazu produzierten Gre2p exprimierende Zellen ausschließlich das (S)-konfigurierte Hydroxyketon **2a**, mit eine hervorragenden Selektivität von >99% (Zeilen 4 bis 6 in Tabelle 2).

**Tabelle 2: Biokatalytische Aktivität der Stämme**

| | MB-STV | LbADH@*E.coli* | | | Gre2p@*E.coli* | | | Produkte [%] | | |
|---|---|---|---|---|---|---|---|---|---|---|
| | | SBP | HOB | ST | SBP | HOB | ST | (R)¹⁾ | (S)²⁾ | SD³⁾ |
| **1** | | x | | | | | | >99 | <1 | <1 |
| **2** | | | x | | | | | >99 | <1 | <1 |
| **3** | | | | x | | | | >99 | <1 | <1 |
| **4** | | | | | x | | | <1 | >99 | <1 |
| **5** | | | | | | x | | <1 | >99 | <1 |
| **6** | | | | | | | x | <1 | >99 | <1 |
| **7** | x | | x | | x | | | 4 | 96 | 4 |
| **8** | x | | x | x | x | | | 6 | 94 | 1 |

| | | | | | | | | | | |
|---|---|---|---|---|---|---|---|---|---|---|
| ¹⁾Summe der (R)-konfigurierten Hydroxyketone **2c/2d** und des Diols **3d**, bestimmt durch chirale HPLC-Analyse ²⁾Nur das (S)-konfigurierte Hydroxyketon **2b** wurde unter den gegebene Reaktionsbedingungen produziert ³⁾Standardabweichung, erhalten aus mindestens zwei unabhängigen Experimenten | | | | | | | | | | |

### Beispiel 6:

Dank der hohen Stereoselektivitäten wurden die katalytischen Proteine auch als Marker für die selektive Selbstimmobilisierung der Bakterien auf magnetischen Mikropartikeln verwendet. Dazu wurden Mischungen der, die Markierungen präsentierenden, KRED@E.coli-Stämme kompetitiv an MB-STV gebunden. Die gebundenen Zellen wurden durch magnetische Trennung geerntet, wachsen gelassen und nachfolgend für die Transformation von NDK 1 verwendet. Chirale HPLC-Analyse zeigte klar, dass die kompetitive Bindung von LbADH@E.coli-HOB und Gre2p@E.coli-SBP nahezu exklusiv zur Bildung des (S)-konfigurierten Hydroxyketons 2a führte (Zeile 7 in Tabelle 2), in Übereinstimmung mit der erwarteten selektiven Bindung von Gre2p@E.coli-SBP an MB-STV. Selbst in einem sehr anspruchsvollen Test, in dem Gre2p@E.coli-SBP, als Zellen mit der geringeren enzymatischen Aktivität (Tabelle 3), mit zwei Stämmen konkurrierte, welche die aktivere LbADH beherbergten (LbADH@E.coli-HOB, LbADH@E.coli-ST, Zeile 8 in Tabelle 2), führte die spezifische Immobilisierung exklusiv zur Bildung des (S)-konfigurierten Hydroxyketons 2a.

**Tabelle 3: Enzymatische Aktivität ganzer Zellen mit NDK 1 als Substrat**

| Stamm | Aktivität¹⁾ [U/OD600] | Aktivität [U/cww] | Aktivität [U/cdw] |
|---|---|---|---|
| LbADH@*E.coli*-SBP | 17,9± 3,5 | 10,6 ± 2,1 | 46,0 ± 9,1 |
| LbADH@*E. coli*-HOB | 17,8± 1,7 | 10,5 ± 1,0 | 45,7 ± 4,5 |
| LbADH@*E.coli*-ST | 19,9± 1,7 | 11,7 ± 1,0 | 51,1 ± 4,5 |
| Gre2p@*E.coli*-SBP | 3,0 ± 0,5 | 1,8 ± 0,3 | 7,7 ± 1,4 |
| Gre2p@*E.coli*-HOB | 2,4 ± 0,4 | 1,4 ± 0,3 | 6,1± 1,1 |
| Gre2p@*E.coli*-ST | 2,6 ± 0,2 | 1,5 ± 0,1 | 6,6 ± 0,5 |

| | | | |
|---|---|---|---|
| ¹⁾Die Aktivität wurde mittels chiraler HPLC bestimmt. Eine Einheit (U) ist als µmol/min definiert. Die gemessenen OD600-Werte wurden auf ein Volumen von 1 L normalisiert. Für die Berechnung der spezifischen Aktivitäten wurden das Nassgewicht der Zellen *(cell wet weight;* cww) und das Trockengewicht der Zellen *(cell dry weight;* cdw) aus den gemessenen OD600-Werten abgeschätzt. | | | |

### Beispiel 7:

Bei der bakteriellen Oberflächenpräsentation von Proteinen sind neben den bereits erwähnten Bindeproteinen besonders biokatalytisch aktive Proteine interessant, um membranimpermeable Substrate außerhalb der Zelle umzusetzen. Aufgrund der umfassenden Anwendungsmöglichkeiten wurde in den vergangenen Jahren vor allem für das Bakterium *Escherichia coli* eine Vielzahl an unterschiedlichen Oberflächenpräsentations-Systemen entwickelt. In allen bekannten Systemen wird das Zielprotein, welches auf der Oberfläche präsentiert werden soll, an ein genetisches Element fusioniert welches direkt den Transport an die Zelloberfläche verursacht. Die damit einhergehenden Probleme bei der Faltung und den posttranslationalen Modifikationen der Proteine führen häufig zu deren proteolytischen Abbau oder Aktivitätsverlust.

Das hier beschriebene System trennt genetisch das mit einer Bindeeinheit versehene Zielprotein vom mit einer Markierungseinheit versehenen Membranprotein. Als Markierungseinheit wurde SC und als Bindeeinheit ST gewählt. Dieser ermöglicht eine *in vivo* Kopplung mittels einer Isopeptidbindung zwischen einem Lysin des SC-getaggten Membranproteins und der Asparaginsäure des ST-getaggten Zielproteins. In Fig. 11 werden verschiedene Kopplungsvarianten des Zielproteins realisiert, um die Oberflächenexponierung zu testen und sie mit der direkten Fusion des Zielproteins an das Membranprotein zu vergleichen. Als Modellprotein diente das Häm und Flavinmononukleotid enthaltende Protein P450 BM3, welches in der industriellen Biotechnologie besonders für seine stereo- und regiospezifische Hydroxylierung von Steroiden genutzt wird. Die Analyse der enzymatischen Aktivität der intakten Zellen nach der Expression der verschiedenen Varianten zeigt eindrucksvoll, dass im Fall des anspruchsvollen Proteins P450 BM3(A74G F87V)-ST, das hier beschriebene System zu deutlich erhöhter enzymatischer Aktivität auf der Zelloberfläche führt. Des Weiteren ermöglicht das hier beschriebene System die Expression der Zielproteine ohne eine, auch transiente, Veränderung am N- oder C-Terminus des Proteins.

### Diskussion:

Zusammenfassend wird hier die Entwicklung von E. coli-Stämmen berichtet, welche an ihrer Oberfläche orthogonale Markierungen für eine Immobilisierung präsentieren und gleichzeitig funktionelle heterologe Proteine in ihrem Cytosol überexprimieren. Basierend auf der hier gezeigten Anwendbarkeit für die stereoselektive Ganzzellbiokatalyse ist die vorliegende Erfindung von größter Wichtigkeit für die Entwicklung nachhaltiger biotechnologischer Verfahren auf Grundlage von lebenden, selbst-immobilisierenden Biokatalysatoren. Weiter können die an der Zelloberfläche präsentierten Markierungseinheiten auch zur Erzeugung von Mustern und/oder Anhäufungen von miteinander in Kontakt stehenden Zellen auf Oberflächen verwendet werden (Fig. 4), um neue Wege in der synthetischen Biologie und Biotechnologie zur Verfügung zu Stellen.

## Patentansprüche

1. Verfahren zur selektiven Bindung von bakteriellen Zellen an einen Bindungspartner, umfassend die Schritte:
(a) Bereitstellen von bakteriellen Zellen, wobei diese bakteriellen Zellen
(i) auf der Zelloberfläche eine orthogonale Markierungseinheit (Tag) exprimieren und
(ii) gleichzeitig ein funktionelles, heterologes Protein exprimieren,
(b) Bereitstellen eines Bindungspartners, der mindestens eine Art von Bindungseinheit (Bindungs-Tag) trägt, welche spezifisch an die Markierungseinheit der bakteriellen Zellen binden kann und
(c) Inkubieren der in Schritt (a) bereitgestellten bakteriellen Zellen mit dem in Schritt (b) bereitgestellten Bindungspartner, wobei die bakteriellen Zellen über ihre Markierungseinheit an die entsprechende Bindungseinheit auf dem Bindungspartner binden,
wobei die Markierungseinheit und die Bindungseinheit so gewählt sind, dass die Markierungseinheit spezifisch und ausschließlich an die Bindungseinheit bindet, und
wobei der Bindungspartner ein Protein oder Proteinkomplex ist, wobei das Protein oder zumindest ein Protein des Proteinkomplexes ein Fusionsprotein mit der Bindungseinheit darstellt, und wobei das als Bindungspartner fungierende Protein das von den zu bindenden bakteriellen Zellen exprimierte funktionelle, heterologe Protein ist.

2. Verfahren nach Anspruch 1, wobei die bakteriellen Zellen *Escherichia coli* Zellen sind.

3. Verfahren nach Anspruch 1 oder Anspruch 2, wobei die Markierungseinheit eines aus Streptavidin-bindendem Peptid *(streptavidin binding peptide;* SBP), Biotin, SpyTag oder SpyCatcher; Halo-Tag, HOB-Tag *(halo-based oligonucleotide binder),* SNAP-Tag, Myc-Tag, FLAG-Tag und ybbR-Tag umfasst.

4. Verfahren nach einem der Ansprüche 1 bis 3, wobei die Bindungseinheit ausgewählt ist aus Streptavidin, eMA *(enhanced monomeric Avidin),* SpyCatcher oder SpyTag, Chlorhexangruppen, Benzylguaningruppen, anti-Myc-Immunglobulinen, anti-FLAG-Immunglobulinen und Coenzym-A-Gruppen.

5. Verfahren nach einem der Ansprüche 1 bis 4, wobei die Bindung der orthogonalen Markierungseinheit an die Bindungseinheit bereits intrazellulär erfolgt.

6. Zellaggregat, umfassend:
(a) bakterielle Zellen, welche mindestens eine Art von Bindungseinheit (Bindungs-Tag) tragen und
(b) bakteriellen Zellen, wobei diese bakteriellen Zellen
(i) auf der Zelloberfläche eine orthogonale Markierungseinheit (Tag) exprimieren und
(ii) gleichzeitig ein funktionelles, heterologes Protein exprimieren,
wobei die bakteriellen Zellen aus (b) mittels spezifischer Bindung ihrer Markierungseinheit an die entsprechende Bindungseinheit auf den bakteriellen Zellen aus (a) an diese gebunden sind,
wobei die bakteriellen Zellen aus (a) identisch mit den bakteriellen Zellen aus (b) sind,
wobei die Markierungseinheit und die Bindungseinheit so gewählt sind, dass die Markierungseinheit spezifisch und ausschließlich an die Bindungseinheit bindet, und
wobei der Bindungspartner ein Protein oder Proteinkomplex ist, wobei das Protein oder zumindest ein Protein des Proteinkomplexes ein Fusionsprotein mit der Bindungseinheit darstellt, und wobei das als Bindungspartner fungierende Protein das von den zu bindenden bakteriellen Zellen exprimierte funktionelle, heterologe Protein ist.

7. Bindungskomplex, umfassend:
(a) Proteine oder Proteinkomplexe, welche mindestens eine Art von Bindungseinheit (Bindungs-Tag) tragen und
(b) bakterielle Zellen, wobei diese bakteriellen Zellen
(i) auf der Zelloberfläche eine orthogonale Markierungseinheit (Tag) exprimieren und
(ii) gleichzeitig ein funktionelles, heterologes Protein exprimieren,
wobei die bakteriellen Zellen mittels spezifischer Bindung ihrer Markierungseinheit an die entsprechende Bindungseinheit auf den Proteinen oder Proteinkomplexen an diese gebunden sind,
wobei die Markierungseinheit und die Bindungseinheit so gewählt sind, dass die Markierungseinheit spezifisch und ausschließlich an die Bindungseinheit bindet, und
wobei der Bindungspartner ein Protein oder Proteinkomplex ist, wobei das Protein oder zumindest ein Protein des Proteinkomplexes ein Fusionsprotein mit der Bindungseinheit darstellt, und wobei das als Bindungspartner fungierende Protein das von den zu bindenden bakteriellen Zellen exprimierte funktionelle, heterologe Protein ist.

8. Verwendung des Zellaggregats nach Anspruch 6, oder des Bindungskomplexes nach Anspruch 7, als Biokatalysator und/oder Biosensor und/oder Bioreaktor.

## Claims

1. A method for the selective binding of bacterial cells to a binding partner, comprising the steps of:
(a) providing bacterial cells, wherein these bacterial cells
(i) express an orthogonal marking unit (tag) on the cell surface and
(ii) simultaneously express a functional, heterologous protein,
(b) providing a binding partner carrying at least one type of binding unit (binding tag) capable of binding specifically to the marking unit of the bacterial cell, and
(c) incubating the bacterial cells provided in step (a) with the binding partner provided in step (b), wherein the bacterial cells bind to the corresponding binding unit on the binding partner via their marking unit,
wherein the marking unit and the binding unit are selected such that the marking unit binds specifically and exclusively to the binding unit, and
wherein the binding partner is a protein or protein complex, wherein the protein, or at least one protein of the protein complex, represents a fusion protein with the binding unit, and wherein the protein functioning as the binding partner is the functional, heterologous protein expressed by the bacterial cells to be bound.

2. The method according to claim 1, wherein the bacterial cells are *Escherichia coli* cells.

3. The method according to claim 1 or claim 2, wherein the marking unit comprises one of streptavidin-binding peptide (SBP), biotin, SpyTag or SpyCatcher; Halo-Tag, HOB-Tag *(halo-based oligonucleotide binder),* SNAP-Tag, Myc-Tag, FLAG-Tag, and ybbR-Tag.

4. The method according to one of claims 1 to 3, wherein the binding unit is selected from streptavidin, eMA *(enhanced monomeric Avidin),* SpyCatcher or SpyTag, chlorohexane groups, benzylguanine groups, anti-Myc immunoglobulins, anti-FLAG immunoglobulins, and coenzyme A groups.

5. The method according to one of claims 1 to 4, wherein binding of the orthogonal marking unit to the binding unit occurs already intracellularly.

6. A cell aggregate comprising:
(a) bacterial cells carrying at least one type of binding unit (binding tag) and
(b) bacterial cells, wherein these bacterial cells
(i) express an orthogonal marking unit (tag) on the cell surface and
(ii) simultaneously express a functional, heterologous protein,
wherein the bacterial cells from (b) are bound to the corresponding binding unit on the bacterial cells from (a) by specific binding of their marking unit to the corresponding binding unit,
wherein the bacterial cells from (a) are identical to the bacterial cells from (b),
wherein the marking unit and the binding unit are selected such that the marking unit binds specifically and exclusively to the binding unit, and
wherein the binding partner is a protein or protein complex, wherein the protein, or at least one protein of the protein complex, represents a fusion protein with the binding unit, and wherein the protein functioning as the binding partner is the functional, heterologous protein expressed by the bacterial cells to be bound.

7. A binding complex, comprising:
(a) proteins or protein complexes carrying at least one type of binding unit (binding tag) and
(b) bacterial cells, wherein these bacterial cells
(i) express an orthogonal marking unit (tag) on the cell surface and
(ii) simultaneously express a functional, heterologous protein,
wherein the bacterial cells are bound to the corresponding binding unit on the proteins or protein complexes by specific binding of their marking unit to the corresponding binding unit,
wherein the marking unit and the binding unit are selected such that the marking unit binds specifically and exclusively to the binding unit, and
wherein the binding partner is a protein or protein complex, wherein the protein, or at least one protein of the protein complex, represents a fusion protein with the binding unit, and wherein the protein functioning as the binding partner is the functional, heterologous protein expressed by the bacterial cells to be bound.

8. A use of the cell aggregate according to claim 6, or of the binding complex according to claim 7, as a biocatalyst and/or biosensor and/or bioreactor.

## Revendications

1. Procédé de liaison sélective de cellules bactériennes à un partenaire de liaison, comprenant les étapes :
(a) mise à disposition de cellules bactériennes, dans lequel ces cellules bactériennes
(i) expriment une unité de marquage orthogonale (marqueur) à la surface cellulaire et
(ii) expriment simultanément une protéine fonctionnelle hétérologue,
(b) mise à disposition d'un partenaire de liaison qui porte au moins un type d'unité de liaison (marqueur de liaison) qui peut se lier spécifiquement à l'unité de marquage des cellules bactériennes et
(c) incubation des cellules bactériennes mises à disposition à l'étape (a) avec le partenaire de liaison mis à disposition à l'étape (b), dans lequel les cellules bactériennes se lient par le biais de leur unité de marquage à l'unité de liaison correspondante sur le partenaire de liaison,
dans lequel l'unité de marquage et l'unité de liaison sont choisies de sorte que l'unité de marquage se lie spécifiquement et exclusivement à l'unité de liaison, et
dans lequel le partenaire de liaison est une protéine ou un complexe protéique, dans lequel la protéine ou au moins une protéine du complexe protéique représente une protéine de fusion avec l'unité de liaison, et dans lequel la protéine fonctionnant comme partenaire de liaison est la protéine fonctionnelle hétérologue exprimée par les cellules bactériennes à lier.

2. Procédé selon la revendication 1, dans lequel les cellules bactériennes sont des cellules *Escherichia coli.*

3. Procédé selon la revendication 1 ou revendication 2, dans lequel l'unité de marquage comprend un peptide se liant à la streptavidine *(streptavidin binding peptide* ; SBP), de la biotine, SpyTag ou SpyCatcher ; Halo-Tag, HOB-Tag *(halo-based oligonucleotide binder),* SNAP-Tag, Myc-Tag, FLAG-Tag et ybbR-Tag.

4. Procédé selon une des revendications 1 à 3, dans lequel l'unité de liaison est sélectionnée parmi la streptavidine, eMA *(enhanced monomeric Avidin),* SpyCatcher ou SpyTag, des groupes chlorohexane, des groupes benzylguanine, des immunoglobulines anti-Myc, des immunoglobulines anti-FLAG et des groupes de coenzyme-A.

5. Procédé selon une des revendications 1 à 4, dans lequel la liaison de l'unité de marquage orthogonale à l'unité de liaison s'effectue déjà par voie intracellulaire.

6. Agrégat cellulaire, comprenant :
(a) des cellules bactériennes qui portent au moins un type d'unité de liaison (marqueur de liaison) et
(b) des cellules bactériennes, dans lequel ces cellules bactériennes
(i) expriment une unité de marquage orthogonale (marqueur) à la surface cellulaire et
(ii) expriment simultanément une protéine fonctionnelle hétérologue,
dans lequel les cellules bactériennes provenant de (b) sont liées au moyen d'une liaison spécifique de leur unité de marquage à l'unité de liaison correspondante sur les cellules bactériennes provenant de (a) à celles-ci,
dans lequel les cellules bactériennes provenant de (a) sont identiques aux cellules bactériennes provenant de (b),
dans lequel l'unité de marquage et l'unité de liaison sont choisies de sorte que l'unité de marquage se lie spécifiquement et exclusivement à l'unité de liaison, et
dans lequel le partenaire de liaison est une protéine ou un complexe protéique, dans lequel la protéine ou au moins une protéine du complexe protéique représente une protéine de fusion avec l'unité de liaison, et dans lequel la protéine fonctionnant comme partenaire de liaison est la protéine fonctionnel hétérologue exprimée par les cellules bactériennes à lier.

7. Complexe de liaison, comprenant :
(a) des protéines ou complexes protéiques qui portent au moins un type d'unité de liaison (marqueur de liaison) et
(b) des cellules bactériennes, dans lequel ces cellules bactériennes
(i) expriment une unité de marquage orthogonale (marqueur) à la surface cellulaire et
(ii) expriment simultanément une protéine fonctionnelle hétérologue,
dans lequel les cellules bactériennes sont liées au moyen d'une liaison spécifique de leur unité de marquage à l'unité de liaison correspondante sur les protéines ou complexes protéiques à celles/ceux-ci,
dans lequel l'unité de marquage et l'unité de liaison sont choisies de sorte que l'unité de marquage se lie spécifiquement et exclusivement à l'unité de liaison, et
dans lequel le partenaire de liaison est une protéine ou un complexe protéique, dans lequel la protéine ou au moins une protéine du complexe protéique représente une protéine de fusion avec l'unité de liaison, et dans lequel la protéine fonctionnant comme partenaire de liaison est la protéine fonctionnel hétérologue exprimée par les cellules bactériennes à lier.

8. Utilisation de l'agrégat cellulaire selon la revendication 6, ou du complexe de liaison selon la revendication 7, en tant que biocatalyseur et/ou biocapteur et/ou bioréacteur.
